# EUROPEAN PATENT SPECIFICATION

(11) **EP 1 303 606 B1**
(45) Date of publication and mention of the grant of the patent: **02.01.2008**
(21) Application number: 01951681.4
(22) Date of filing: 16.07.2001
(51) Int. Cl.: C12N 15/12, C12N 15/62, C12N 15/63, C07K 14/47, C07K 14/72

(54) **REGULATION OF GENE EXPRESSION USING SINGLE-CHAIN, MONOMERIC, LIGAND DEPENDENT POLYPEPTIDE SWITCHES**
REGULATION DER GENEXPRESSION UNTER VERWENDUNG VON EINKETTIGEN, MONOMEREN, LIGANDEN-ABHÄNGIGEN POLYPEPTIDSCHALTERN
REGULATION D'EXPRESSION GENIQUE A L'AIDE DE COMMUTATEURS POLYPEPTIDIQUES MONOMERES A CHAINE UNIQUE DEPENDANT D'UN LIGAND

(30) Priority: 18.07.2000 US 619063
(43) Date of publication of application: 23.04.2003
(60) Divisional application: 07019198.6
(73) Proprietor: Novartis AG, 4056 Basel (CH); Novartis Pharma GmbH, 1230 Wien (AT); THE SCRIPPS RESEARCH INSTITUTE, La Jolla, CA 92037 (US)
(72) Inventor: BEERLI, Roger, CH-8106 Adlikon (CH); SCHOPFER, Ulrich, 79539 Lörrach (DE); BARBAS, Carlos, F., Solana Beach, CA 92075 (US)
(74) Representative: Winter, Brandl, Fürniss, Hübner Röss, Kaiser, Polte Partnerschaft Patent- und Rechtsanwaltskanzlei
(86) International application number: PCT/EP2001/008190
(87) International publication number: WO 2002/006463

(56) References cited:
- EP-A- 0 540 065
- WO-A-00/23464
- WO-A-01/30843
- WO-A-98/18925
- WO-A-99/10510
- WO-A-99/47656
- WO-A2-01/36447
- BEERLI ROGER R ET AL: "Chemically regulated zinc finger transcription factors." JOURNAL OF BIOLOGICAL CHEMISTRY, vol. 275, no. 42, 20 October 2000 (2000-10-20), pages 32617-32627, XP002199787 ISSN: 0021-9258
- BEERLI ROGER R ET AL: "Positive and negative regulation of endogenous genes by designed transcription factors" PROCEEDINGS OF THE NATIONAL ACADEMY OF SCIENCES OF USA, NATIONAL ACADEMY OF SCIENCE. WASHINGTON, US, vol. 97, no. 4, 15 February 2000 (2000-02-15), pages 1495-1500, XP002188512 ISSN: 0027-8424
- BURCIN M M ET AL: "A REGULATORY SYSTEM FOR TARGET GENE EXPRESSION" FRONTIERS IN BIOSCIENCE, XX, XX, vol. 3, 1 March 1998 (1998-03-01), pages 1-7, XP000874619
- WHITELAW M.L. ET AL: 'Definition of a novel ligand binding domain of a nuclear bHLH receptor: co-localization of ligand and hsp90 binding activities within the regulable inactivation domain of the dioxin receptor' EMBO J. vol. 12, no. 11, 1993, pages 4169 - 4179
- WHITELAW M.L. ET AL: 'Identification of Transactivation and Repression Functions of the Dioxin Receptor and its Basic Helix-Loop-Helix/PAS Partner Factor Arnt: Inducible versus Constitutive Modes of Regulation' MOLECULAR AND CELLULAR BIOLOGY vol. 14, no. 12, December 1994, pages 8343 - 8355
- SCHOENMAKER E. ET AL: 'Differentical DNA binding by the androgen and glucocorticoid receptors involves a second Zn-finger and a C-terminal extension of the DNA-binding domains' BIOCHEMICAL JOURNAL vol. 341, 1999, pages 515 - 521
- BRZOZOWSKI ET AL: 'Molecular basis of agonism and antagonism in the oestrogen receptor' NATURE vol. 389, 16 October 1997, pages 753 - 758
- CHAMBRAUD ET AL: 'Several Regions of Human Estrogen Receptor Are Involved in the Formation of Receptor-Heat Shock Protein 90 Complexes' J. BIOL. CHEM. vol. 265, no. 33, 25 November 1990, pages 20686 - 20691

## Description

### Technical Field of the Invention

The field of this invention is regulation of transcription. More particularly, the present invention pertains to polypeptides that can activate or repress transcription in a small molecule ligand-dependent manner.

### Background of the Invention

Designed transcription factors with defined target specificity and regulatory function provide invaluable tools for basic and applied research, and for gene therapy. Accordingly, the design of sequence-specific DNA binding domains has been the subject of intense interest for the last two decades. Of the many classes of DNA binding proteins studied, the modular Cys2 -His2 zinc finger DNA binding motif has shown the most promise for the production of proteins with tailored DNA binding specificity. The novel architecture of this class of proteins provides for the rapid construction of gene-specific targeting devices. Polydactyl zinc finger proteins are most readily prepared by assembly of modular zinc finger domains recognizing predefined three-nucleotide sequences (See, e.g., Segal, D. J., Dreier, B., Beerli, R. R., and Barbas, C. F., III (1999) Proc. Natl. Acad. Sci. USA 96, 2758-2763; Beerli, R. R., Segal, D. J., Dreier, B., and Barbas, C. F., III (1998) Proc. Natl. Acad. Sci. USA 95, 14628-14633; and Beerli, R. R., Dreier, B., and Barbas, C. F., III (2000) Proc. Natl. Acad Sci. USA 97, 1495-1500)*.* Polydactyl proteins can be assembled using variable numbers of zinc finger domains of varied specificity providing DNA binding proteins that not only recognize novel sequences but also sequences of varied length. By combining six zinc finger domains, proteins have been produced that recognize 18 contiguous base pairs of DNA sequence, a DNA address sufficiently complex to specify any locus in the 4 billion-base pair human genome (or any other genome). Fusion of polydactyl zinc finger proteins of this type to activation or repression domains provides transcription factors that efficiently and specifically modulate the expression of both transgenes and endogenous genes (Beerli, R R., Segal, D. J., Dreier, B., and Barbas, C. F., III (1998) Proc. Natl. Acad. Sci. USA. 95, 14628-14633; and Beerli, R. R., Dreier, B., and Barbas, C. F., III (2000) Proc. Natl. Acad. Sci. USA 97, 1495-1500).

While the availability of designed transcription factors with tailored DNA binding specificities provides novel opportunities in transcriptional regulation, additional applications would be available to ligand-dependent transcription factors. Designer zinc finger proteins dependent on small molecule inducers would have a number of applications, both for the regulation of endogenous genes, and for the development of inducible expression systems for the regulation of transgenes. Natural transcription factors are regulated by a number of different mechanisms, including postranslational modification such as phosphorylation (Janknecht, R., and Hunter, T. (1997) EMBO J 16,1620-1627; Darnell, J. E., Jr. (1997) Science 277, 1630-1635), or by ligand binding. The prototype ligand-activated transcription factors are members of the nuclear hormone receptor family, including the receptors for sex steroids or adrenocorticoids (Carson-Jurica, M. A., Schrader, W. T., and O'Malley, W. (1990) Endocrine Reviews 11, 201-220; Evans, R. M. (1988) Science 240, 889-895). These receptors are held inactive in the absence of hormone, by association with a number of inactivating factors including hsp90 (Pratt, W. B., and Toft, D. O. (1997) Endocrine Rev. 18, 306-360). Upon ligand binding, nuclear hormone receptors dissociate from the inactivating complex, dimerize, and become able to bind DNA and activate transcription (Carson-Jurica, M. A., Schrader, W. T., and O'Malley, W. (1990) Endocrine Reviews 11, 201-220; Evans, R. M. (1988) Science 240, 889-89512-14; and Pratt, W. B., and Toft, D. O. (1997) Endocrine Rev. 18, 306-360). Significantly, not only hormone binding but also inactivation and dimerization functions reside within the ligand binding domain (LBD) of these proteins (Beato, M. (1989) Cell 56, 335-344). This fact has been exploited experimentally and steroid hormone receptor LBDs have found wide use as tools to render heterologous proteins hormone-dependent.

In particular, the estrogen receptor (ER) LBD has been used to render the functions of c-Myc (Eilers, M., Picard, D., Yamamoto, K. R., and Bishop, J. M. (1989) Nature 340, 66-68), c-Fos (Superti-Furga, G., Bergers, G., Picard, D., and Busslinger, M. (1991) Proc. NatL Acad Sci. USA 88, 5114-5118), and even the cytoplasmic kinase c-Raf (. Samuels, M. L., Weber, M. J., Bishop, J. M., and McMahon, M. (1993) Mol. Cell. Biol. 13, 6241-6252) hormone-dependent. To develop an inducible expression system for use in basic research and gene therapy, the availability of ligand-dependent transcriptional regulators is a prerequisite. Preferentially, these regulators would be activated by a small molecule inducer with no other biological activity, bind specific sequences present only in the target promoter, and have low immunogenicity. A number of ligand-regulated artificial transcription factors have been generated by various means, using functional domains derived from either prokaryotes (Gossen, M., and Bujard, H. (1992) Proc. Natl. Acad Sci. USA 89, 5547-5551 20. Gossen, M., Freundlieb, S., Bender, G., Müller, G., Hillen, W., and Bujard. H. (1995) Science 268, 1766-1769 21. Labow, M. A., Baim, S. B., Shenk, T., and Levine, A. J. (1990) Mol. Cell. Biol. 10, 3343-3356 22. Baim, S. B., Labow, M. A., Levine, A. J., and Shenk, T. (1991) Proc. Natl. Acad. Sci. USA 88, 5072-5076) or eukaryotes (Christopherson, K. S., Mark, M. R., Bajaj, V., and Godowski, P. J. (1992) Proc. Natl. Acad. Sci. USA 89, 6314-6318 24. No, D., Yao, T.-P., and Evans, R. M. (1996) Proc. Natl. Acad Sci. USA 93, 3346-3351 25. Wang, Y., O'Malley, B. W., Jr., Tsai, S., and O'Malley, B. W. (1994) Proc. Natl. Acad. Sci. USA 91, 8180-8184 Beerli *et al.* -35-26. Wang, Y., Xu, J., Pierson, T., O'Malley, B. W., and Tsai, S. Y. (1997) Gene Therapy 4, 432-441 27. Braselmann, S., Graninger, P., and Busslinger, M. (1993) Proc. Natl. Acad Sci. USA 90, 1657-1661 28. Louvion, J. F., Havaux-Copf, B., and Picard, D. (1993) Gene 131, 129-134.29. Rivera. V. M.,Clackson, T.Natesan. S., Pollock, R., Amara, J. F., Keenan, T., Magari, S. R., Phillips, T., Courage, N. L., Cerasoli, F., Jr., Holt, D. A., and Gilman, M. (1996) Nature. Med 2, 1028-1032).

Of the functional domains derived from eukaryotic proteins, nuclear hormone receptor LBDs have been the most widely used. Whitelaw M. L et al., EMBO J. 12 (1993), 4169-4179 and Whitelaw M.L. et al., MCB 14, (1994),8343-8355 disclose chimaeras between the nuclear receptors for glucocorticoids and dioxin.

WO-A-O1/36447, which is prior art under Art. 54(3) EPC, discloses chimeric proteins having at least two functional protein units, wherein each unit comprises the dimerization domain of a member of the steroid/thyroid hormone nuclear receptor superfamily. Each unit may contain a ligand binding domain, a DNA binding domain and an activation domain. In particular, regulators based on the Gal4 DNA binding domain (DBD) fused to a human ER (Braselmann, S., Graninger, P., and Busslinger, M. (1993) Proc. Natl. Acad. Sci. USA 90,1657-1661; Louvion, J. F., Havaux-Copf, B., and Picard, D. (1993) Gene 131,129-134) or progesterone receptor (PR) LBD; (Wang, Y., O'Malley, B. W., Jr., Tsai, S., and O'Malley, B. W. (1994) Proc. Natl. Acad Sci. USA 91, 8180-8184; Wang, Y., Xu, J., Pierson, T., O'Malley, B. W., and Tsai, S. Y. (1997) Gene Therapy 4, 432-441), as well as the ecdysone-inducible system based on the *Drosophila* ecdysone receptor (EcR) and the mammalian retinoid X receptor (RXR) (Christopherson, K. S., Mark, M. R, Bajaj, V., and Godowski, P. J. (1992) Proc. Natl. Acad. Sci. USA 89, 6314-6318; No, D., Yao, T.-P., and Evans, R. M. (1996) Proc. Natl. Acad. Sci. USA 93, 3346-3351) have been described. Compared to the heterodimeric EcR/RXR system, regulators based on the ER and PR LBDs have the important advantage that they function as homodimers and require the delivery of only one cDNA. However, while ecdysone has no known biological effect on mammalian cells, estrogen and progesterone will elicit a biological response in cells or tissues that express the endogenous steroid receptors. With the availability of a mutated ER and a truncated PR LBDs that have lost responsiveness to their natural ligands but not to synthetic antagonists such as 4-hydroxytamoxifen (4-OHT) (Littlewood, T. D., Hancock, D. C., Danielian, P. S., Parker, M. G., and Evan, G. L (1995) Nucl. Acids Res. 23, 1686-1690) or RU486 (Vegeto, E., Allan, G. F., Schrader, W. T., Tsai, M.-J., McDonnell, D. P., and O'Malley, B. W. (1992) Cell 69, 703-713), respectively, this is no longer of great concern. Thus, steroid hormone receptor LBD-based inducible expression systems can be developed that function independently of the endogenous steroid receptors. To date, this has been shown for the PR LBD through the development of an RU486-inducible expression system based on the Gal4 DBD (Wang, Y., O'Malley, B. W., Jr., Tsai, S., and O'Malley, B. W. (1994) Proc. Natl. Acad. Sci. USA 91, 8180-8184; Wang, Y., Xu, J., Pierson, T., O'Malley, B. W., and Tsai, S. Y. (1997) Gene Therapy 4, 432-441). An inducible expression system based on a point-mutated (G525R) ER LBD (Littlewood, T. D., Hancock, D. C., Danielian, P. S., Parker, M. G., and Evan, G. L (1995) Nucl. Acids Res. 23, 1686-1690) that has lost the responsiveness to estrogen but not the antagonist 4-OHT has not been described to date. Designed zinc finger proteins have a number of advantages as compared to other DBDs, including the one derived from Gal4, since the ability to engineer DNA binding specificities allows ligand-dependent regulators to be directed to any desired artificial or natural promoter. Here we explore the utility of fusion proteins between designed zinc finger proteins and nuclear hormone receptor LBDs for the inducible control of gene expression.

### Brief Summary of the Invention

The present invention provides a non-naturally occurring polypeptide that contains two ligand binding domains operatively linked to each other, a first functional domain which is a DNA binding domain operatively linked to one of the ligand binding domains, and a second functional domain which is a transcriptional regulation domain. The ligand binding domains are preferably covalently linked to each other. More preferably, the two binding domains are covalently linked by means of a peptide linker that contains from about 10 to about 40 amino acid residues, preferably from about 15 to about 35 amino acid residues and, more preferably from about 18 to about 30 amino acid residues.

The ligand binding domains are derived from nuclear hormone receptors. The ligand binding domains can be derived from the same or different nuclear hormone receptors. Exemplary and preferred nuclear hormone receptors are steroid hormone receptors such as an estrogen receptor, a progesterone receptor, an ecdysone receptor and a retinoid X, receptor.

The first functional domain alters the function or activity of a target nucleotide and is a DNA binding domain. The DNA binding domain preferably contains at least one engineered zinc finger DNA binding motif, more preferably from two to twelve zinc finger DNA binding motifs and, even more preferably from three to six zinc finger DNA binding motifs. In one embodiment, the zinc finger DNA binding motifs specifically bind to a nucleotide sequence of the formula (GNN)₁₋₆, where G is guanidine and N is any nucleotide. In another embodiment, the first functional The second functional domain is a transcriptional regulating domain such as a transcription activation domain or a transcription repression domain.

In one embodiment, a polypeptide of this invention includes (a) a DNA binding domain having from three to six zinc finger DNA binding motifs; (b) a first ligand binding domain derived from a retinoid X receptor operatively linked to the DNA binding domain, a second ligand binding domain derived from an eedyzone receptor linked to the first ligand binding domain with a peptide spacer of from 18 to 36 amino acid residues; and (c) a transcription regulating domain operatively linked to the second binding domain.

In still another embodiment, a polypeptide gene switch includes (a) a DNA binding domain having from three to six zinc finger DNA binding motifs; (b) a first ligand binding domain derived from a progesterone receptor operatively linked to the DNA binding domain, a second ligand binding domain derived from a progesterone receptor linked to the first ligand binding domain with a peptide spacer of from 18 to 36 amino acid residues; and (c) a transcription regulating domain operatively linked to the second ligand binding domain.

In another aspect, the present invention provides polynucleotides that encode a polypeptide gene switch of the invention, expression vectors containing such polynucleotides and cells containing such nucleotides.

Another aspect of this invention provides a process of regulating the function of a target nucleotide that contains a defined sequence. The process includes the step of exposing in vitro or ex vivo the target nucleotide to a polypeptide of this invention in the presence of a ligand that binds at least one of the ligand binding domains of the polypeptide. In a related aspect, the present invention provides a process for regulating transcription (e.g., expression) of a target nucleotide (e.g., gene). In accordance with that process a target nucleotide that contains a defined sequence is exposed to a polypeptide of this invention in the presence of a ligand that binds to at least one of the ligand binding domains of that polypeptide. The polypeptide contains a DNA binding domain that specifically binds to the defined sequence in the target nucleotide. Where the polypeptide gene switch contains a transcription repression domain, regulating is repression. Where the polypeptide gene switch contains a transcription activation domain, regulating is activation.

### Brief Description of the Drawings

In the drawings that form a portion of the specification

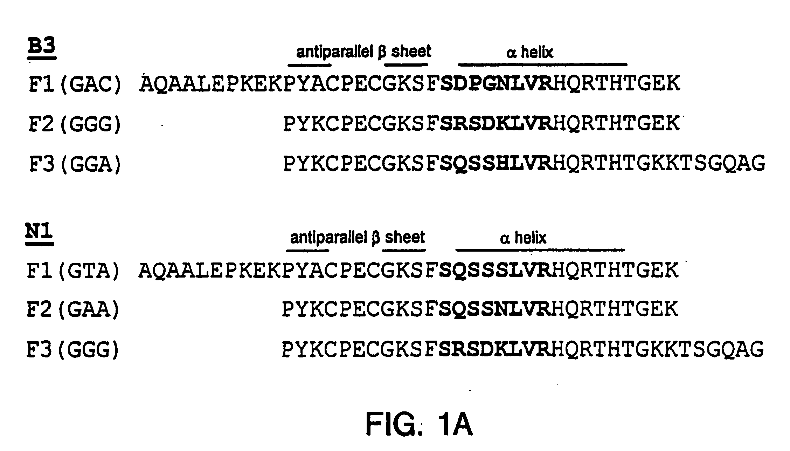

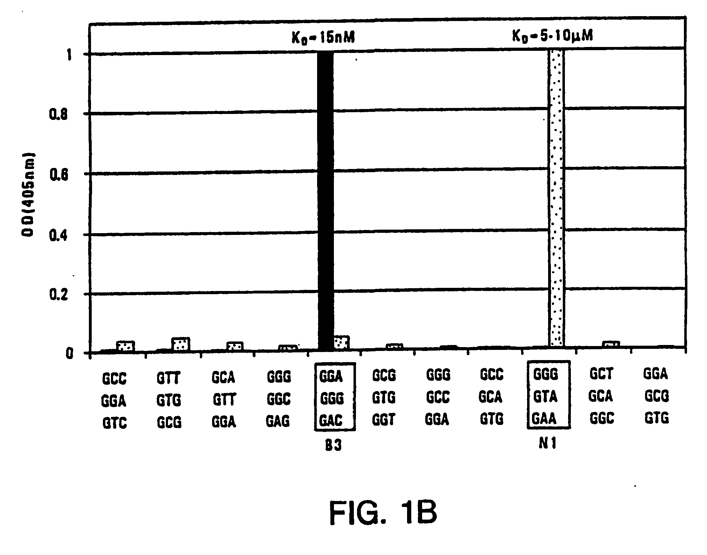

**Figure 1** shows generation of designed zinc finger proteins with novel DNA binding specificity. *A*, amino acid sequence of the three-finger proteins B3 and N1. DNA recognition helix positions -2 to 6, shown in bold print, were grafted into the framework of the three finger protein Sp1C. The location of the antiparallel β sheets and the α helices, structural hallmarks of zinc finger domains, are as indicated. DNA binding specificity of each finger is show on the left. F1-3, Finger 1-3. *B*, ELISA analysis of DNA binding specificity. Zinc finger proteins were expressed in *E. coli* as MBP fusions and purified. Specificity of binding was analyzed by measuring binding to immobilized biotinylated hairpin oligonucleotides containing the indicated 5'-(GNN)₃-3'sequences. Black bars, B3; gray bars, N1. The maximal signals were normalized to 1. The K_{D} value for binding to the specific target sequence was measured by electrophoretic mobility shift assay and is labeled on top of the corresponding bars.

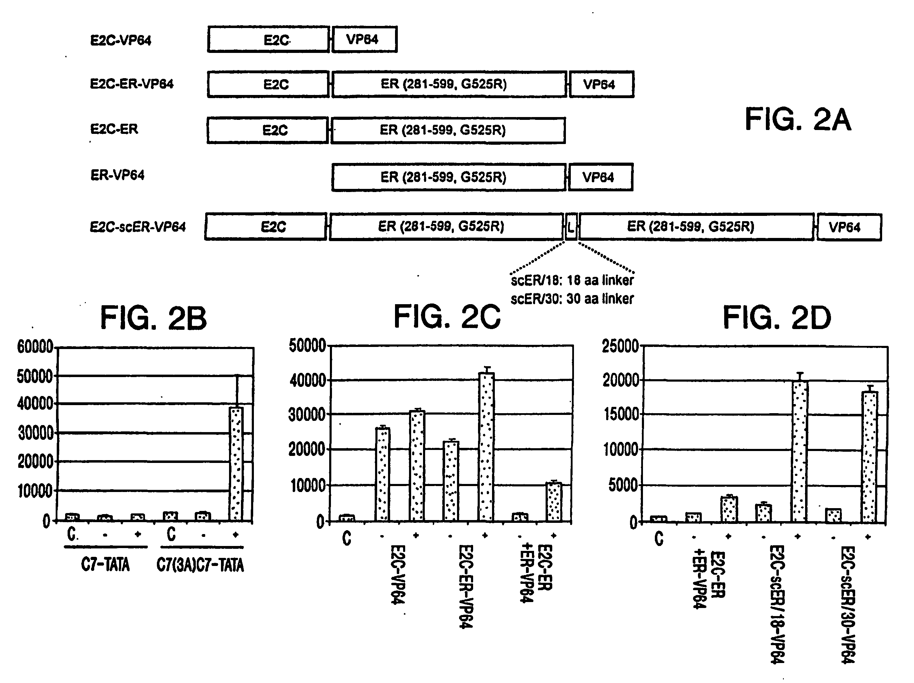

**Figure 2** shows regulation of gene expression by hormone-dependent, single-chain ER fusion constructs. *A*, structure of ER fusion proteins. E2C, six finger protein; L, flexible peptide linker. *B*, fusion proteins with a single ER-LBD bind as dimers. HeLa cells were cotransfected with a C7-ER-VP64 expression vector, and the indicated TATA luciferase reporter plasmids carrying either one or two C7 binding sites. 24 b after transfection, cells were either left untreated (-), or 100 nM 4-OHT was added (+). Luciferase activity in total cell extracts was measured 48 h after transfection. Each bar represents the mean value (+/- SD) of duplicate measurements. C, control plasmid pcDNA3 that does not express a fusion protein. *C, D,*-regulation of transcription through a single binding site by fusion proteins with two ER-LBDs. HeLa cells were cotransfected with the indicated expression vectors and the E2C-TATA-luciferase reporter plasmid, carrying a single E2C binding site upstream of a TATA box. 4-OHT induction and measurement of luciferase activity was carried out as described in *B*.

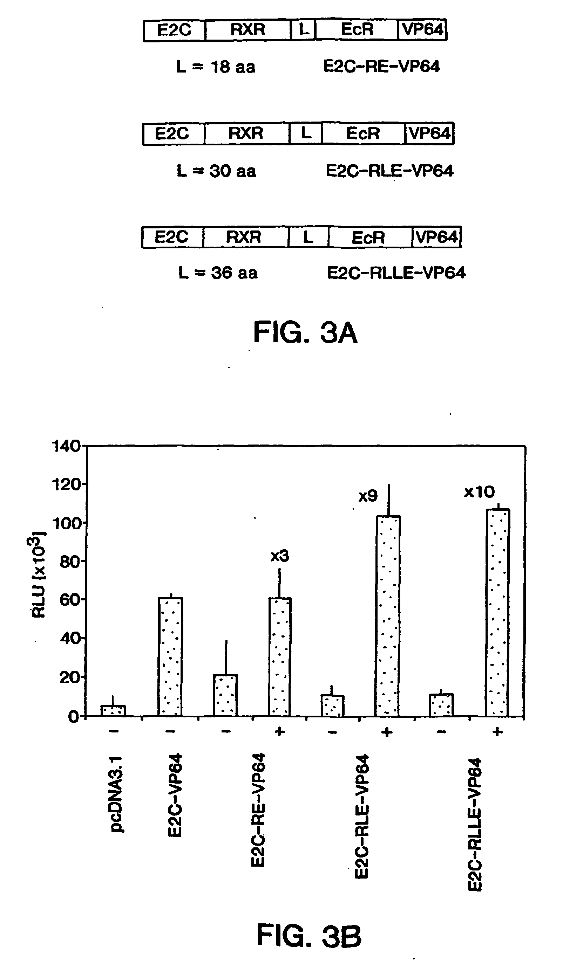

**Figure 3** shows regulation of gene expression by hormone-dependent, single-chain RXR/EcR fusion constructs. *A*, structure of single-chain RXR/EcR fusion proteins. *B,* regulation of transcription through a single binding site. HeLa cells were cotransfected with the indicated expression vectors and the E2C-TATA-luciferase reporter plasmid, carrying a single E2C binding site. 24 h after transfection, cells were either left untreated (-), or 5 µM Ponasterone A was added (+). Luciferase activity in total cell extracts was measured 48 h after transfection. Each bar represents the mean value (+/- SD) of duplicate measurements. pcDNA3.1, control plasmid that does not express a fusion protein.

**Figure 4** shows the nucleotide (SEQ ID NO: 31) and amino acid residue sequence (SEQ ID NO: 32) of zinc finger binding domain B3B.

**Figure 5** shows the nucleotide (SEQ ID NO: 33) and amino acid residue sequence (SEQ ID NO: 34) of zinc finger binding domain 2C7.

**Figure 6** shows the nucleotide (SEQ ID NO: 35) and amino acid residue sequence (SEQ ID NO: 36) of zinc finger binding domain B3C2.

**Figure 7** shows the nucleotide (SEQ ID NO: 37) sequence of repression domain (KRAB-A)₂.

**Figure 8** shows the nucleotide (SEQ ID NO: 38) sequence of repression domain (SID)₂.

**Figure 9** shows the nucleotide (SEQ ID NO: 39) and amino acid residue sequence (SEQ ID NO: 40) of polypeptide E2C-ER-L-ER-VP64.

**Figure 10** shows the nucleotide (SEQ ID NO: 41) and amino acid residue sequence (SEQ ID NO: 42) of polypeptide E2C-ER-LL-ER-VP64.

### Detailed Description of the Invention

### I. The Invention

The present invention provides polypeptide gene switches, polynucleotides that encode such polypeptides, expression vectors that contain such polynucleotides, cells that contain such expression vectors or polynucleotides and processes for regulating target nucleotide function using such polypeptides, polynucleotides and expression vectors. Unlike existing gene switches that contain a single ligand binding domain together with a DNA binding domain and/or a transcriptional regulating domain, polypeptide gene switches of the present invention contain two ligand binding domains. Upon binding of the ligand, an intramolecular configuration change occurs that allows for alignment of the functional domains to the target gene of interest. An advantage of the present gene switches, therefore, over existing gene switches is the need for only a single molecular switch and a single expression vector for production of that switch.

### II. Polypeptides

A polypeptide gene switch of the present invention includes two ligand binding domains (LBDs) and a first functional domain (FD-1). The ligand binding domains are operatively linked to the first functional domain such that the polypeptide, in the presence of a defined ligand that binds to at least one of the ligand binding domains, can alter the function of nucleotide. The domains can be arranged in any order. As shown below, the ligand binding domains can be situated in either the amino-or carboxyl-terminal direction from the first functional domain.

| | |
|---|---|
| LBDs | FD-1 |

| | |
|---|---|
| FD-1 | LBDs |

A polypeptide of this invention is non-naturally occurring. As used herein, the term "non-naturally occurring" means, for example, one or more of the following: (a) a peptide comprised of a non-naturally occurring amino acid sequence; (b) a peptide having a non-naturally occurring secondary structure not associated with the peptide as it occurs in nature; (c) a peptide which includes one or more amino acids not normally associated with the species of organism in which that peptide occurs in nature; (d) a peptide which includes a stereoisomer of one or more of the amino acids comprising the peptide, which stereoisomer is not associated with the peptide as it occurs in nature; (e) a peptide which includes one or more chemical moieties other than one of the natural amino acids; or (f) an isolated portion of a naturally occurring amino acid sequence (e.g., a truncated sequence). A polypeptide of this invention exists in an isolated form and purified to be substantially free of contaminating substances. A polypeptide is synthetic in nature. That is, the polypeptide is isolated and purified from natural sources or made *de novo* using techniques well known in the art.

### A. Ligand Binding Domain (LBD)

Each LBD is an amino acid residue sequence that is capable of and binds a particular ligand. Binding of the ligand to the LBD alters the conformation/function of the polypeptide and allows for regulating a function of a target nucleotide. In the absence of ligand, the gene switch does not work to alter nucleotide function. At least one of the LBDs is capable of binding and binds a particular ligand. Both LBDs can bind a particular ligand. Thus, the LBDs can be the same or different. Preferred LBDs are derived from nuclear hormone receptors such as steroid hormone receptors.

Exemplary and preferred steroid receptors that can serve as the source of ligand binding domains include the estrogen receptor (ER), progesterone receptor (PR), glucocorticoid-α receptor, glucocorticoid-β receptor, mineralocorticoid receptor, androgen receptor, thyroid hormone receptor, retinoic acid receptor (RAR), retinoid X receptor (RXR), Vitamin D receptor, COUP-TF receptor, ecdysone receptor (EcR), Nurr-1 receptor and orphan receptors. A preferred EcR is derived either from *Drosophila melanogaster* (DE) or *Bombyx* (BE).

As is well known in the art, steroid hormone are composed of a DNA binding domain and a ligand binding domain. The DNA binding domain contains the receptor regulating sequence and binds DNA and the ligand binding domain binds the specific biological compound (ligand) to activate the receptor. The term "ligand" refers to any compound which activates the receptor, usually by interaction with (binding) the ligand binding domain of the receptor. However, ligands also include compounds that activate the receptor without binding. Where used in a polypeptide gene switch of the present invention, it is preferred that the ligand receptor domain be modified from its naturally occurring ligand, a ligand other than the naturally occurring ligand (e.g. steroid hormone). Means of altering or derivatizing naturally occurring nuclear hormone receptor ligand binding domains to alter the binding specificity are well known in the art (See, e.g. United States Patent Nos. 5,874,534 and 5,599,904). Similarly, means for altering the estrogen receptor to change its bind affinity have reported [See, e.g. Littlewood et al., Nucleic Acids Res., 3(10):1686-1690,1995].

The term "naturally occurring ligand" refers to compounds that are normally not found in animals or humans and which bind to the ligand binding domain of a receptor. The ligand can also be a "non-native ligand", a ligand that is not naturally found in the specific organism (man or animal) in which gene therapy is contemplated. For example, certain insect hormones such as ecdysone are not found in humans. This is an example of a non-native hormone to the animal or human.

Examples of non-natural ligands, anti-hormones and non-native ligands include the following: 11β-(4-dimethylaminophenyl)-17β-hydroxy-17α-propinyl-4,9-e stradiene-3-one (Ru38486 or Mifepestone); 11β-(4-dimethylaminophenyl)-17α-hydroxy-17β-(3-hydroxypropyl)-13α-methyl-4,9-gonadiene-3-one (ZK98299 or Onapristone); 11β-(4-acetylphenyl)-17β-hydroxy-17α-(1-propinyl)-4,9-estradiene-3-one (ZK112993); 11β-(4-dimenthylaminophenyl)-17β-hydroxy-17α-(3-hydroxy-1(Z)-propenyl-estra-4,9-diene-3-one (ZK98734); (7β11β17β)-11-(4-dimethylaminophenyl)-7-methyl-4',5' dihydrospiroy' ester-4,9-diene-17,2'(3'H)-furan!-3-one (Org31806): (11β,14β,17α)-4',5'-dihydro-11-(4-dimethylaminophenyl)y'spi rostra-4,9-diene-17,2'(3,H)-furan!-3-one (Org31376); 5-alpha-pregnane-3,2-dione. Additional non-natucal ligands include, in general, synthetic non-steroidal estrogenic or anti-estrogenic compounds, broadly defined as selective estrogen receptor modulators (SERMS). Exemplary compounds include, but are not limited to, tamoxifen and raloxifen,

Exemplary and preferred ligands for use with various ligand binding domains are (1) EcR: Ponasterone a, Muristerone A, GS-E (Invitrogen), Tebufenocide; (2) ER: estrogen antagonists such as 4-hydroxy-tamoxifen, ICI 164384, RU 54876, Raloxifene; and (3) PR: progesterone antagonists such as RU 486, RU 38486, and Onapristone.

An especially preferred LBD derived from a progesterone receptor comprises amino acid residues 645-914 from the human progesterone receptor. An exemplary LBD derived from an estrogen receptor comprises amino acid residues 282-599 from the mouse G225R mutant.

The two LBDs are separated be an amino acid residue sequence linker that contains from about 10 to about 50 amino acid residues. Preferably, the spacer contains from about 15 to about 40 amino acid residues and, more preferably, from about 18 to about 35 amino acid residues. Exemplary and preferred spacers contain 18 (L), 30 (LL), or 36 (LLL) amino acid residues.

### B. Functional Domains

A second component of a present polypeptide is a functional domain. As used herein, the term "functional domain" and it's grammatical equivalents, means an amino acid residue sequence that binds to, alter the structure of, and/or alters the function of, a nucleotide. Exemplary such functional domains include nucleotide binding domains, transcriptional regulating domains (e.g. transcription activation domains and transcription repression domains) and domains having nuclease activity. Such domains are well known in the art.

### 1. Nucleotide Binding Domains

A functional domain of a polypeptide can be a DNA binding domain, a sequence of amino acid residues that recognize and bind to a defined DNA sequence. Amino acid residue sequences that recognize and bind to defined DNA sequences are well known in the art (e.g., GAL4). The DNA binding domain of a polypeptide gene switch of this invention comprises one or more engineered DNA binding zinc finger motifs. Such zinc finger DNA binding motifs are well known in the art (See. e.g., PCT Patent Application Nos. WO95/19421 and WO 98/54311).

A DNA binding domain of a polypeptide gene switch of this invention, thus, preferably includes a multiple finger, polydactyl, zinc finger peptide that is designed to bind specific nucleotide target sequences.

The present disclosure is based on the recognition of the structural features unique to the Cys₂-His₂ zinc finger domain consist of a simple ββα fold of approximately 30 amino acids in length. Structural stability of this fold is achieved by hydrophobic interactions and by chelation of a single zinc ion by the conserved Cys₂-His₂ residues (Lee, M.S., Gippert, G.P., Soman, K.V., Case, D.A. & Wright, P.E. (1989) Science 245, 635-637). Nucleic acid recognition is achieved through specific amino acid side chain contacts originating from the α-helix of the domain, which typically binds three base pairs of DNA sequence (Pavletich, N. P. & Pabo, C.O. (1991) Science 252, 809-17, Elrod-Erickson, M., Rould, M.A., Nekludova, L. & Pabo, C.O. (1996) Structure 4, 1171-1180). Unlike other nucleic acid recognition motifs, simple covalent linkage of multiple zinc finger domains allows the recognition of extended asymmetric sequences of DNA.

Studies of natural zinc finger proteins have shown that three zinc finger domains can bind 9 bp of contiguous DNA sequence (Pavletich, N.P. & Pabo, C.O. (1991) Science 252, 809-17., Swimoff, A.H. & Milbrandt, J. (1995) Mol. Cell. Biol. 15, 2275-87). Whereas recognition of 9 bp of sequence is insufficient to specify a unique site within even the small genome of *E.coli,* polydactyl proteins containing six zinc fingers domains can specify 18-bp recognition (Liu, Q., Segal, DJ., Ghiara, J.B. & Barbas III, C.F. (1997) Proc. Natl. Acad Sci. USA 94, 5525-5530). With respect to the development of a universal system for gene control, and 18-bp address can be sufficient to specify a single site within all known genomes. And their efficacy in gene activation and repression within living human cells has recently been shown (Liu, Q., Segal, DJ., Ghiara, J.B. & Barbas III, C.F. (1997) Proc. Natl. Acad. Sci. USA 94, 5525-5530).

The engineered zinc finger-nucleotide binding peptide domain can be derived or produced from a wild type zinc finger protein by truncation or expansion, or as a variant of the wild type-derived polypeptide by a process of site directed mutagenesis, or by a combination of the procedures. The term "truncated" refers to a zinc finger-nucleotide binding polypeptide that contains less that the full number of zinc fingers found in the native zinc finger binding protein or that has been deleted of non-desired sequences. For example, truncation of the zinc finger-nucleotide binding protein TFIIIA. which naturally contains nine zinc fingers, might be a polypeptide with only zinc fingers one through three. Expansion refers to a zinc finger polypeptide to which additional zinc finger modules have been added. For example, TFIIIA may be extended to 12 fingers by adding 3 zinc finger modules from more than one wild type polypeptide, thus resulting in a "hybrid" zinc finger-nucleotide binding polypeptide.

The term "mutagenized" refers to a zinc finger derived-nucleotide binding polypeptide that has been obtained by performing any of the known methods for accomplishing random or site-directed mutagenesis of the DNA encoding proteins. For instance, in TFIIIA, mutagenesis can be preformed to replace non-conserved residues in one or more of the repeats of the consensus sequence. Truncated zinc finger-nucleotide binding proteins can also be mutagenized. Examples of known zinc finger-nucleotide binding proteins can also be mutagenized. Examples of known zinc finger-nucleotide binding polypeptides that can be truncated, expanded, and/or mutagenized according to the present invention in order to inhibit the function of a nucleotide sequence containing a zinc finger-nucleotide binding motif includes TFIIIA and zif268. Other zinc finger-nucleotide binding proteins will be known to those of skill in the art.

A zinc finger DNA binding domain can be made using a variety of standard techniques well known in the art. Phage display libraries of zinc finger proteins were created and selected under conditions that favored enrichment of sequence specific proteins. Zinc finger domains recognizing a number of sequences required refinement by site-directed mutagenesis that was guided by both phage selection data and structural information.

A DNA binding domain used in a polypeptide of this invention is preferably a zinc finger-nucleotide binding peptide that binds to a (GNN)₁₋₆ nucleotide sequence. Zinc fingers that bind specifically to (GNN)₁₋₆ have been described in United States Patent Application Serial Number 09/173,941, filed October 16,1998.

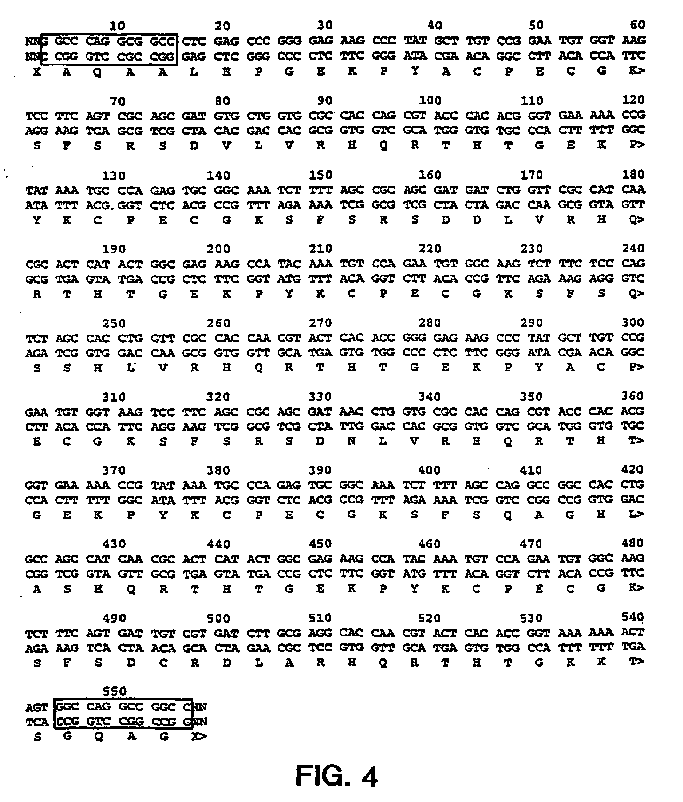

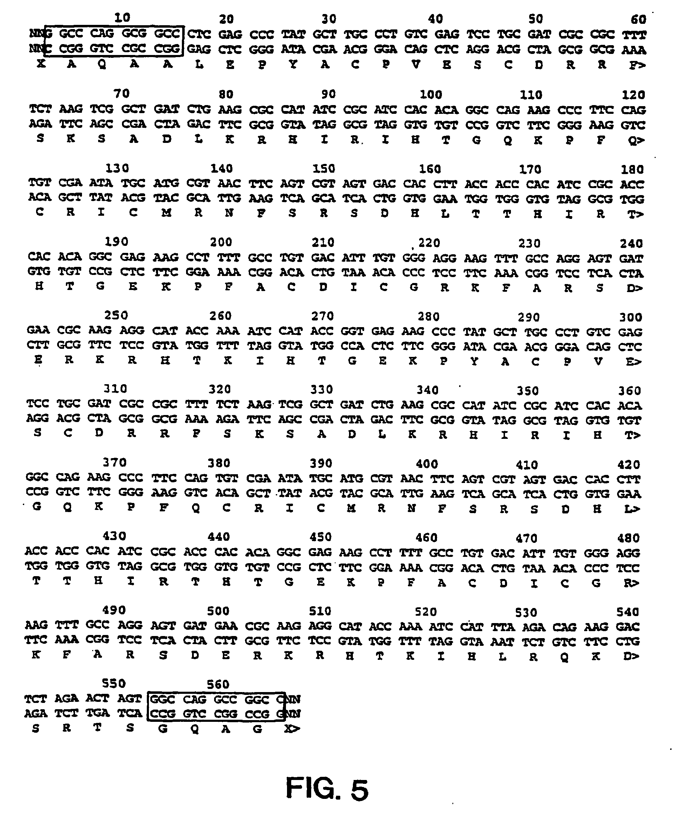

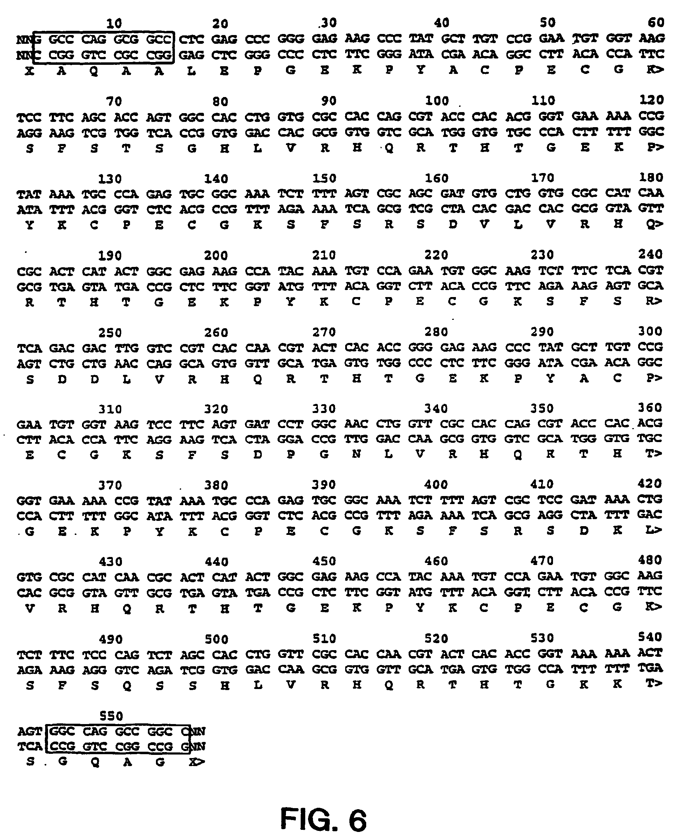

Exemplary and preferred zinc finger DNA binding domains are designated herein as E2C, C7, B3B, 2C7, B3C2 and N1. A detailed description of the preparation of polypeptide gene switches containing zinc finger DNA binding domains can be found hereinafter in the Examples. The amino acid residue and encoding nucleotide sequences for B3B, 2C7 and B3C2 are shown in FIGs. 4-6, respectively.

### 2. Transcription Regulating Domains

A transcription regulating domain refers to a peptide, which acts to activate or repress transcription of a target nucleotide (e.g., gene). Transcriptional activation domains are well known in the art (See. e.g., Seipel et al., (1992) EMBO J., 11.4961-4969). Exemplary and preferred transcription activation domains include VP16, TA2, VP64, STAT6, relA, TAF-1, TAF-2, TAU-1 and TAU-2. Especially preferred activation domains for use in the present invention are VP16 and VP64. Means for linking VP16 and VP64 to ligand binding domains are set forth hereinafter in the Examples.

Transcriptional repressor domains are also well known in the art. Exemplary and preferred such transcriptional repressors are ERD, KRAB, SID, histone deacetylase, DNA, methylase, and derivatives, multimers and combinations thereof such as KRAB-ERD, SID-ERD, (ERAB)₂, (KRAB)₃, KRAB-A, (KRAB-A)₂, (SID)₂, (KRAB-A)-SID and SID-(KRAB-A). A first repressor domain can be prepared using the Krηppel-associated box (KRAB) domain (Margolin *et al.,* 1994). This repressor domain is commonly found at the N-terminus of zinc finger proteins and presumably exerts its repressive activity on TATA-dependent transcription in a distance- and orientation-independent manner, by interacting with the RING finger protein KAP-1. One can utilize the KRAB domain found between amino acids 1 and 97 of the zinc finger protein KOX1. Finally, to explore the utility of histone deacetylation for repression, amino acids 1 to 36 of the Mad mSIN2 interaction domain (SID) can be fused to another domain (Ayer *et al.,* 1996). This small domain is found at the N-terminus of the transcription factor Mad and is responsible for mediating its transcriptional repression by interacting with mSIN3, which in turn interacts the co-repressor N-CoR and with the histone deacetylase mRPD1.

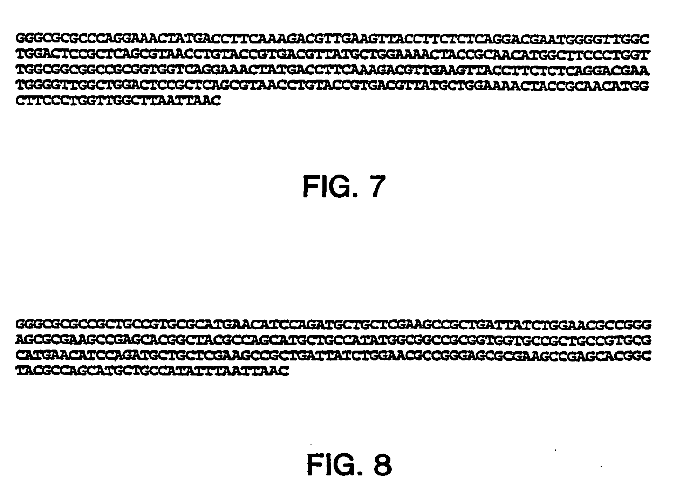

The amino acid residue and nucleotide encoding sequences of preferred transcriptional repression domains (KRAB-A)2 and (SID)₂ are shown in FIGs 7 and 8, respectively. Means for linking repression domains to ligand binding domains as well as exemplary polypeptide gene switches containing repression domains are set forth hereinafter in the Examples.

### 3. Polypeptide Gene Switches

A polypeptide of this invention comprises two ligand binding domains, a first functional domain and a second functional domain. These domains can exist in any order as shown below.

In a preferred embodiment the two ligand binding domains (LBDs) are located directly adjacent to one another, ie. they are "serially connected" within the monomeric polypeptide gene switch of the invention and are not separated by a functional domain of the invention. The serially connected LBDs may be separated from one another by a linker molecule, such as for example a polypeptide linker molecule.

In a preferred embodiment the two LBDs are located between two functional domains (FDs) of the invention, wherein one functional domain is a Transcription Regulating Domain (TRD) and the other functional domain is a Nucleotide Binding Domain (NBD).

In one particularly preferred embodiment the monomeric polypeptide gene switch of the invention consists of two FDs and two LBDs in the sequential order FD-1 / LBD-1 / LBD-2 / FD-2. In this embodiment, one functional domain is a TRD and the other functional domain is a NBD.

Preferredly, the NBD employed in the monomeric polypeptide gene switch of the invention includes 6 zinc finger binding motifs. As further described in the examples hereinbelow, a 6 zinc finger NBD employed in a monomeric polypeptide gene switch allows for the recognition of a unique 18bp nucleic acid sequence, which may be symmetric or asymmetric.

| | | |
|---|---|---|
| LBDs | FD-1 | FD-2 |

| | | |
|---|---|---|
| FD-1 | FD-2 | I LBDs |

| | | |
|---|---|---|
| FD-1 | LBDs | FD-2 |
| | | |
| FD-2 | LBDs | FD-1 |
| | | |
| FD-2 | FD-1 | LBDs |

A wide variety of polypeptide gene switches have been made. Exemplary such gene switches include (see above for definition of terms):

### Gene Switches Using RXR, E2C, and Activation Domains

E2C-RXR-L-DE-VP64, E2C-RXR-LL-DE-VP64, EZC-RXR-LLL-DE-VP64, E2C-RXR-L-BE-VP64, E2C-RXR-LL-BE-VP64, E2C-RXR-LLL-BE-VP64, E2C-RXR-L-DE-VP16, E2C-RXR-LL-DE-VP16, E2C-RXR-LLL-DE-VP16, E2C-RXR-L-BE-VP16, E2C-RXR-LL-BE-VP16, E2C-RXR-LLL-BE-VP16;

### Gene Switches Using RXR, 2C7, and Activation Domains

2C7-RXR-L-DE-VP64,2C7-RXR-LL-DE-VP64,2C7-RXR-LLL-DE-VP64, 2C7-RXR-L-BE-VP64, 2C7-RXR-LL-BE-VP64, 2C7-RXR-LLL-BE-VP64,2C7-RXR-L-DE-VP16,2C7-RXR-LL-DE-VP16,2C7-RXR-LLL-DE-VP16, 2C7-RXR-L-BE-VP16, 2C7-RXR-LL-BE-VP16, E2C-RXR-LLL-BE-VP16;

### Gene Switches Using RXR, B3B, and Activation Domains

B3B-RXR-L-DE-VP64, B3B-RXR-LL-DE-VP64, B3B-RXR-LLL-DE-VP64, B3B 7-RXR-L-BE-VP64, B3B 7-RXR-LL-BE-VP64, B3B-RXR-LLL-BE-VP64, B3B-RXR-L-DE-VP16, B3B-RXR-LL-DE-VP16, B3B-RXR-LLL-DE-VP16, B3B-RXR-L-BE-VP16, B3B-RXR-LL-BE-VP16, B3B-RXR-LLL-BE-VP16;

### Gene Switches Using RXR, B3C2, and Activation Domains

B3C2-RXR-L-DE-VP64, B3C2-RXR-LL-DE-VP64, B3C2-RXR-LLL-DE-VP64, B3C2-RXR-L-BE-VP64, B3C2-RXR-LL-BE-VP64, B3C2-RXR-LLL-BE-VP64, B3C2-RXR-L-DE-VP16, B3C2-RXR-LL-DE-VP16, B3C2-RXR-LLL-DE-VP16, B3C2-RXR-L-BE-VP16, B3C2 B-RXR-LL-BE-VP16, B3C2-RXR-LLL-BE-VP16;

### Gene Switches Using RXR, E2C. and Repression Domains

E2C-RXR-L-DE-(KRAB-A)2, E2C-RXR-LL-DE-(KRAB-A)2, E2C-RXR-LLL-DE-(KRAB-A)2, E2C-RXR-L-BE-(KRAB-A)2, E2C-RXR-LL-BE-(KRAB-A)2, E2C-RXR-LLL-BE-(KRAB-A)2, E2C-RXR-L-DE-(KRAB-A)2, E2C-RXR-LL-DE-(KRAB-A)2, E2C-RXR-LLL-DE-(KRAB-A)2, E2C-RXR-L-BE-(KRAB-A)2, E2C-RXR-LL-BE-(KRAB-A)2, E2C-RXR-LLL-BE-(KRAB-A)2, E2C-RXR-L-DE-(SID)2, E2C-RXR-LL-DE-(SID)2, E2C-RXR-LLL-DE-(SID)2, E2C-RXR-L-BE-(SID)2, E2C-RXR-LL-BE-(SID)2, E2C-RXR-LLL-BE-(SID)2, E2C-RXR-L-DE-(SID)2, E2C-RXR-LL-DE-(SID)2, E2C-RXR-LLL-DE-(SID)2, E2C-RXR-L-BE-(SID)2, E2C-RXR-LL-BE-(SID)2, E2C-RXR-LLL-BE-(SID)2;

### Gene Switches Using RXR, 2C7, and Repression Domains

2C7-RXR-L-DE-(KRAB-A)2, 2C7-RXR-LL-DE-(KRAB-A)2, 2C7-RXR-LLL-DE-(KRAB-A)2, 2C7-RXR-L-BE-(KRAB-A)2, 2C7-RXR-LL-BE-(KRAB-A)2, 2C7-RXR-LLL-BE-(KRAB-A)2, 2C7-RXR-L-DE-(KRAB-A)2, 2C7-RXR-LL-DE-(KRAB-A)2, 2C7-RXR-LLL-DE-(KRAB-A)2, 2C7-RXR-L-BE-(KRAB-A)2,2C7-RXR-LL-BE-(KRAB-A)2, E2C-RXR-LLL-BE-(KRAB-A)2, 2C7-RXR-L-DE-(SID)2,2C7-RXR-LL-DE-(SID)2,2C7-RXR-LLL-DE-(SID)2,2C7-RXR-L-BE-(SID)2,2C7-RXR-LL-BE-(SID)2,2C7-RXR-LLL-BE-(SID)2,2C7-RXR-L-DE-(SID)2,2C7-RXR-LL-DE-(SID)2,2C7-RXR-LLL-DE-(SID)2,2C7-RXR-L-BE-(SID)2,2C7-RXR-IL-BE-(SID)2, EZC-RXR-LLL-BE-(SID)2,n;

### Gene Switches Using RXR, B3B. and Repression Domains

B3B-RXR-L-DE-(KRAB-A)2, B3B-RXR-LL-DE-(KRAB-A)2, B3B-RXR-LLL-DE-(IGtAB-A)2, B3B 7-RXR-L-BE-(KRAB-A)2, B3B 7-RXR-LL-BE-(KRAB-A)2, B3B-RXR-LLL-BE-(KRAB-A)2, B3B-RXR-L-DE-(KRAB-A)2, B3B-RXR-LL-DE-(KRAB-A)2, B3B-RXR-LLL-DE-(KRAB-A)2, B3B-RXR-L-BE-(KRAB-A)2, B3B-RXR-LL-BE-(KRAB-A)2, B3B-RXR-LLL-BE-(KRAB-A)2, B3B-RXR-L-DE-(SID)2, B3B-RXR-LL-DE-(SID)2, B3B-RXR-LLL-DE-(SID)2, B3B 7-RXR-L-BE-(SID)2, B3B 7-RXR-LL-BE-(SID)2, B3B-RXR-LLL-BE-(SID)2, B3B-RXR-L-DE-(SID)2, B3B-RXR-LL-DE-(SID)2, B3B-RXR-LLL-DE-(SID)2, B3B-RXR-L-BE-(SID)2, B3B-RXR-LL-BE-(SID)2, B3B-RXR-LLL-BE-(SID)2;

### Gene Switches Using RXR, B3C2, and Repression Domains,

B3C2-RXR-L-DE-(KRAB-A)2, B3C2-RXR-LL-DE-(KRAB-A)2, B3C2-RXR-LLL-DE-(KRAB-A)2, B3C2-RXR-L-BE-(KRAB-A)2, B3C2-RXR-LL-BE-(KRAB-A)2, B3C2-RXR-LLL-BE-(KRAB-A)2, B3C2-RXR-L-DE-(KRAB-A)2, B3C2-RXR-LL-DE-(KRAB-A)2, B3C2-RXR-LLL-DE-(KRAB-A)2, B3C2-RXR-L-BE-(KRAB-A)2, B3C2 B-RXR-LL-BE-(KRAB-A)2, B3C2-RXR-LLL-BE-(KRAB-A)2, B3C2-RXR-L-DE-(SID)2, B3C2-RXR-LL-DE-(SID)2, B3C2-RXR-LLL-DE-(SID)2, B3C2-RXR-L-BE-(SID)2, B3C2-RXR-LL-BE-(SID)2, B3C2-RXR-LLL-BE-(SID)2, B3C2-RXR-L-DE-(SID)2, B3C2-RXR-LL-DE-(SID)2, B3C2-RXR-LLL-DE-(SID)2, B3C2-RXR-L-BE-(SID)2, B3C2 B-RXR-LL-BE-(SID)2, B3C2-RXR-LLL-BE-(SID)2;

### Gene Switches Using PR, E2C, and Activation Domains

E2C-PR-L-PR-VP64, E2C-PR-LL-PR-VP64, E2C-PR-LLL-PR-VP64, E2C-PR-L-PR-VP64, E2C-PR-LL-PR-VP64, E2C-PR-LLL-PR-VP64, E2C-PR-L-PR-VP16, E2C-PR-LL-PR-VP16, E2C-PR-LLL-PR-VP16, E2C-PR-L-PR-VP16, E2C-PR-LL-PR-VP16, E2C-PR-LLL-PR-VP16;

### Gene Switches Using PR, 2C7, and Activation Domains

2C7-PR-L-PR-VP64, 2C7-PR-LL-PR-VP64, 2C7-PR-LLL-PR-VP64, 2C7-PR-L-PR-VP64, 2C7-PR-LL-PR-VP64, 2C7-PR-LLL-PR-VP64, 2C7-PR-L-PR-VP16, 2C7-PR-LL-PR-VP16, 2C7-PR-LLL-PR-VP16, 2C7-PR-L-PR-VP16, 2C7-PR-LL-PR-VP16, E2C-PR-LLL-PR-VP16;

### Gene Switches Using PR, B3B, and Activation Domains

B3B-PR-L-PR-VP64, B3B-PR-LL-PR-VP64, B3B-PR-LLL-PR-VP64, B3B 7-PR-L-PR-VP64, B3B 7-PR-LL-PR-VP64, B3B-PR-LLL-PR-VP64, B3B-PR-L-PR-VP16, B3B-PR-LL-PR-VP16, B3B-PR-LLL-PR-VP16, B3B-PR-L-PR-VP16, B3B-PR-LL-PR-VP16, B3B-PR-LLL-PR-VP16;

### Gene Switches Using PR, B3C2, and Activation Domains

B3C2-PR-L-PR-VP64, B3C2-PR-LL-PR-VP64, B3C2-PR-LLL-PR-VP64, B3C2-PR-L-PR-VP64, B3C2-PR-LL-PR-VP64, B3C2-PR-LLL-PR-VP64, B3C2-PR-L-PR-VP16, B3C2-PR-LL-PR-VP16, B3C2-PR-LLL-PR-VP16, B3C2-PR-L-PR-VP16, B3C2 B-PR-LL-PR-VP16, B3C2-PR-LLL-PR-VP16;

### Gene Switches Using PR, E2C, and Repression Domains

E2C-PR-L-PR-(KRAB-A)2, E2C-PR-LL-PR-(KRAB-A)2, E2C-PR-LLL-PR-(KRAB-A)2, E2C-PR-L-PR-(KRAB-A)2, E2C-PR-LL-PR-(KRAB-A)2, E2C-PR-LLL-PR-(KRAB-A)2, E2C-PR-L-PR-(KRAB-A)2, E2C-PR-LL-PR-(KRAB-A)2, E2C-PR-LLL-PR-(KRAB-A)2, E2C-PR-L-PR-(KRAB-A)2, E2C-PR-LL-PR-(KRAB-A)2, E2C-PR-LLL-PR-(KRAB-A)2, E2C-PR-L-PR-(SID)2, E2C-PR-LL-PR-(SID)2, E2C-PR-LLL-PR-(SID)2, E2C-PR-L-PR-(SID)2, E2C-PR-LL-PR-(SID)2, E2C-PR-LLL-PR-(SID)2, E2C-PR-L-PR-(SID)2, E2C-PR-LL-PR-(SID)2, E2C-PR-LLL-PR-(SID)2, E2C-PR-L-PR-(SID)2, E2C-PR-LL-PR-(SID)2, E2C-PR-LLL-PR-(SID)2;

### Gene Switches Using PR. 2C7, and Repression Domains

2C7-PR-L-PR-(KRAB-A)2,2C7-PR-LL-PR-(KRAB-A)2,2C7-PR-LLL-PR-(KRAB-A)2,2C7-PR-L-PR-(KRAB-A)2,2C7-PR-LL-PR-(KRAB-A)2,2C7-PR-LLL PR-(KRAB-A)2,2C7-PR-L-PR-(KRAB-A)2, 2C7-PR-LL-PR-(KRAB-A)2,2C7-PR-LLL-PR-(KRAB-A)2,2C7-PR-L-PR-(KRAB-A)2,2C7-PR-LL-PR-(KRAB-A)2, E2C-PR-LLL-PR-(KRAB-A)2, 2C7-PR-L-PR-(SID)2,2C7-PR-LL-PR-(SID)2, 2C7-PR-LLL-PR-(SID)2,2C7-PR-L-PR-(SID)2,2C7-PR-LL-PR-(SID)2,2C7-PR-LLL-PR-(SID)2,2C7-PR-L-PR-(SID)2,2C7-PR-LL-PR-(SID)2,2C7-PR-LLL-PR-(SID)2,2C7-PR-L-PR-(SID)2,2C7-PR-LL-PR-(SID)2, E2C-PR-LLL-PR-(SID)2,n;

### Gene Switches Using PR, B3B. and Repression Domains

B3B-PR-L-PR-(KRAB-A)2, B3B-PR-LL-PR-(KRAB-A)2, B3B-PR-LLL-PR-(KRAB-A)2, B3B 7-PR-L-PR-(KRAB-A)2, B3B 7-PR-LL-PR-(KRAB-A)2, B3B-PR-LLL-PR-(KRAB-A)2, B3B-PR-L-PR-(KRAB-A)2, B3B-PR-LL-PR-(KRAB-A)2, B3B-PR-LLL-PR-(KRAB-A)2, B3B-PR-L-PR-(KRAB-A)2, B3B-PR-LL-PR-(KRAB-A)2, B3B-PR-LLL-PR-(KRAB-A)2, B3B-PR-L-PR-(SID)2, B3B-PR-LL-PR-(SID)2, B3B-PR-LLL-PR-(SID)2, B3B 7-PR-L-PR-(SID)2, B3B 7-PR-LL-PR-(SID)2, B3B-PR-LLL-PR-(SID)2, B3B-PR-L-PR-(SID)2, B3B-PR-LL-PR-(SID)2, B3B-PR-LLL-PR-(SID)2, B3B-PR-L-PR-(SID)2, B3B-PR-LL-PR-(SID)2, B3B-PR-LLL-PR-(SID)2;

### Gene Switches Using PR. B3C2, and Repression Domains

B3C2-PR-L-PR-(KRAB-A)2, B3C2-PR-LL-PR-(KRAB-A)2, B3C2-PR-LLL-PR-(KRAB-A)2, B3C2-PR-L-PR-(KRAB-A)2, B3C2-PR-LL-PR-(KRAB-A)2, B3C2-PR-LLL-PR-(KRAB-A)2, B3C2-PR-L-PR-(KRAB-A)2, B3C2-PR-LL-PR-(KRAB-A)2, B3C2-PR-LLL-PR-(KRAB-A)2, B3C2-PR-L-PR-(KRAB-A)2, B3C2 B-PR-LL-PR-(KRAB-A)2, B3C2-PR-LLL-PR-(KRAB-A)2, B3C2-PR-L-PR-(SID)2, B3C2-PR-LL-PR-(SID)2, B3C2-PR-LLL-PR-(SID)2, B3C2-PR-L-PR-(SID)2, B3C2-PR-LL-PR-(SID)2, B3C2-PR-LLL-PR-(SID)2, B3C2-PR-L-PR-(SID)2, B3C2-PR-LL-PR-(Sm)2, B3C2-PR-LLL-PR-(SID)2, B3C2-PR-L-PR-(SID)2, B3C2 B-PR-LL-PR-(SID)2, B3C2-PR-LLL-PR-(SID)2;

### Gene Switches Using ER, E2C, and Activation Domains

E2C-ER-L-ER-VP64, E2C-ER-LL-ER-VP64, E2C-ER-LLL-ER-VP64, E2C-ER-L-ER-VP64, E2C-ER-LL-ER-VP64, E2C-ER-LLL-ER-VP64, E2C-ER-L-ER-VP16, EZC-ER-LL-ER-VP16, E2C-ER-LLL-ER-VP16, E2C-ER-L-ER-VP16, E2C-ER-LL-ER-VP16, E2C-ER-LLL-ER-VP16;

### Gene Switches Using ER, 2C7, and Activation Domains

2C7-ER-L-ER-VP64, 2C7-ER-LL-ER-VP64, 2C7-ER-LLL-ER-VP64, 2C7-ER-L-ER-VP64,2C7-ER-LL-ER-VP64,2C7-ER-LLL-ER-VP64,2C7-ER-L-ER-VP16, 2C7-ER-LL-ER-VP16, 2C7-ER-LLL-ER-VP16, 2C7-ER-L-ER-VP16, 2C7-ER-LL-ER-VP16, E2C-ER-LLL-ER-VP16;

### Gene Switches Using ER, B3B, and Activation Domains

B3B-ER-L-ER-VP64, B3B-ER-LL-ER-VP64, B3B-ER-LLL-ER-VP64, B3B 7-ER-L-ER-VP64, B3B 7-ER-LL-ER-VP64, B3B-ER-LLL-ER-VP64, B3B-ER-L-ER-VP16, B3B-ER-LL-ER-VP16, B3B-ER-LLL-ER-VP16, B3B-ER-L-ER-VP16, B3B-ER-LL-ER-VP16, B3B-ER-LLL-ER-VP16;

### Gene Switches Using ER, B3C2, and Activation Domains

B3C2-ER-L-ER-VP64, B3C2-ER-LL-ER-VP64, B3C2-ER-LLL-ER-VP64, B3C2-ER-L-ER-VP64, B3C2-ER-LL-ER-VP64, B3C2-ER-LLL-ER-VP64, B3C2-ER-L-ER-VP16, B3C2-ER-LL-ER-VP16, B3C2-ER-LLL-ER-VP16, B3C2-ER-L-ER-VP16, B3C2 B-ER-LL-ER-VP16, B3C2-ER-LLL-ER-VP16;

### Gene Switches Using ER, E2C, and Repression Domains

E2C-ER-L-ER-(KRAB-A)2, E2C-ER-LL-ER-(KRAB-A)2, E2C-ER-LLL-ER-(KRAB-A)2, E2C-ER-L-ER-(KRAB-A)2, E2C-ER-LL-ER-(KRAB-A)2, E2C-ER-LLL-ER-(KRAB-A)2, E2C-ER-L-ER-(KRAB-A)2, E2C-ER-LL-ER-(KRAB-A)2, E2C-ER-LLL-ER-(KRAB-A)2, E2C-ER-L-ER-(KRAB-A)2, E2C-ER-LL-ER-(KRAB-A)2, E2C-ER-LLL-ER-(KRAB-A)2, E2C-ER-L-ER-(SID)2, E2C-ER-LL-ER-(SID)2, E2C-ER-LLL-ER-(SID)2, E2C-ER-L-ER-(SID)2, E2C-ER-LL-ER-(SID)2, E2C-ER-LLL-ER-(SID)2, E2C-ER-L-ER-(SID)2, E2C-ER-LL-ER-(SID)2, E2C-ER-LLL-ER-(SID)2, E2C-ER-L-ER-(SID)2, E2C-ER-LL-ER-(SID)2, E2C-ER-LLL-ER-(SID)2;

### Gene Switches Using ER, 2C7, and Repression Domains

2C7-ER-L-ER-(KRAB-A)2, 2C7-ER-LL-ER-(KRAB-A)2, 2C7-ER-LLL-ER-(KRAB-A)2, 2C7-ER-L-ER-(KRAB-A)2, 2C7-ER-LL-ER-(KRAB-A)2, 2C7-ER-LLL-ER-(KRAB-A)2, 2C7-ER-L-ER-(KRAB-A)2, 2C7-ER-LL-ER-(KRAB-A)2, 2C7-ER-LLL-ER-(KRAB-A)2, 2C7-ER-L-ER-(KRAB-A)2,2C7-ER-LL-ER-(KRAB-A)2, E2C-ER-LLL-ER-(KRAB-A)2, 2C7-ER-L-ER-(SID)2, 2C7-ER-LL-ER-(SID)2,2C7-ER-LLL-ER-(SID)2,2C7-ER-L-ER-(SID)2,2C7-ER-LL-ER-(SID)2,2C7-ER-LLL-ER-(SID)2,2C7-ER-L-ER-(SID)2,2C7-ER-LL-ER-(SID)2,2C7-ER-LLL-ER-(SID)2,2C7-ER-L-ER-(SID)2,2C7-ER-LL-ER-(SID)2, E2C-ER-LLL-ER-(SID)2,n;

### Gene Switches Using ER. B3B, and Repression Domains

B3B-ER-L-ER-(KRAB-A)2, B3B-ER-LL-ER-(KRAB-A)2, B3B-ER-LLL-ER-(KRAB-A)2, B3B 7-ER-L-ER-(KRAB-A)2, B3B 7-ER-LL-ER-(KRAB-A)2, B3B-ER LLL-ER-(IGtAB-A)2, B3B-ER-L-ER-(KRAB-A)2, B3B ER-LL-ER-(KRAB-A)2, B3B-ER-LLL-ER-(KRAB-A)2, B3B-ER-L-ER-(IGtAB-A)2, B3B-ER-LL-ER-(KRAB-A)2, B3B-ER-LLL-ER-(KRAB-A)2, B3B-ER-L-ER-(SID)2, B3B-ER-LL-ER-(SID)2, B3B-ER-LLL-ER-(SID)2, B3B 7-ER-L-ER-(SID)2, B3B 7-ER-LL-ER-(SID)2, B3B-ER-LLL-ER-(SID)2, B3B-ER-L-ER-(SID)2, B3B-ER-LL-ER-(SID)2, B3B-ER-LLL-ER-(SID)2, B3B-ER-L-ER-(SID)2, B3B-ER-LL-ER-(SID)2, B3B-ER-LLL-ER-(SID)2;

### Gene Switches Using ER, B3C2. and Repression Domains

B3C2-ER-L-ER-(KRAB-A)2, B3C2-ER-LL-ER-(KRAB-A)2, B3C2-ER-LLL-ER-(KRAB-A)2, B3C2-ER-L-ER-(KRAB-A)2, B3C2-ER-LL-ER-(KRAB-A)2, B3C2-ER-LLL-ER-(KRAB-A)2, B3C2-ER-L-ER-(KRAB-A)2, B3C2-ER-LL-ER-(KRAB-A)2, B3C2-ER-LLL-ER-(KRAB-A)2, B3C2-ER-L-ER-(KRAB-A)2, B3C2 B-ER-LL-ER-(KRAB-A)2, B3C2-ER-LLL-ER-(KRAB-A)2, B3C2-ER-L-ER-(SID)2, B3C2-ER-LL-ER-(SID)2, B3C2-ER-LLL-ER-(SID)2, B3C2-ER-L-ER-(SID)2, B3C2-ER-LL-ER-(SID)2, B3C2-ER-LLL-ER-(SID)2, B3C2-ER-L-ER-(SID)2, B3C2-ER-LL-ER-(SID)2, B3C2-ER-LLL-ER-(SID)2, B3C2-ER-L-ER-(SID)2, B3C2 B-ER-LL-ER-(SID)2, B3C2-ER-LLL-ER-(SID)2.

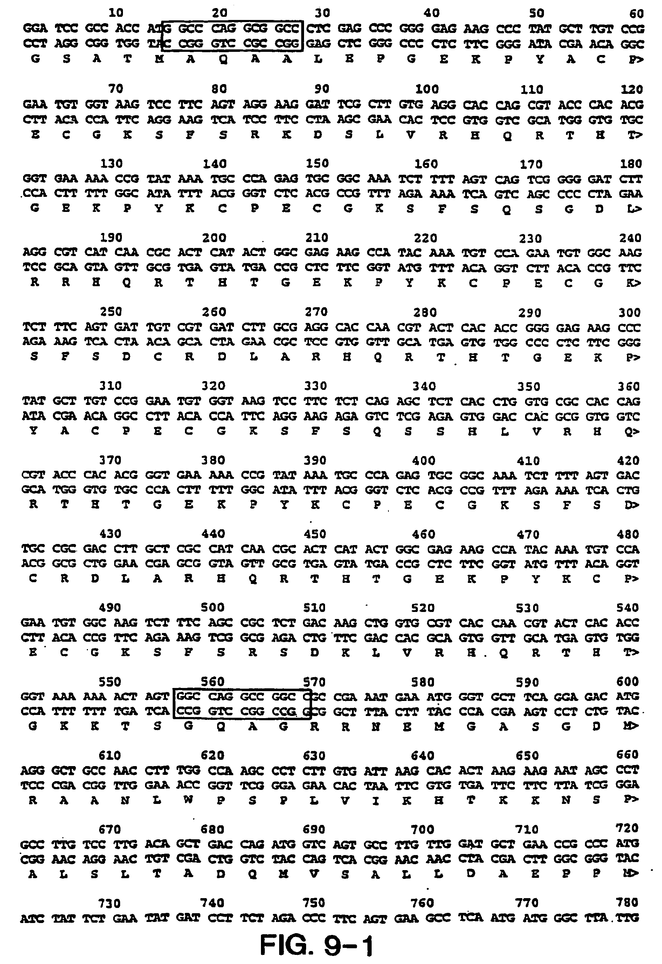

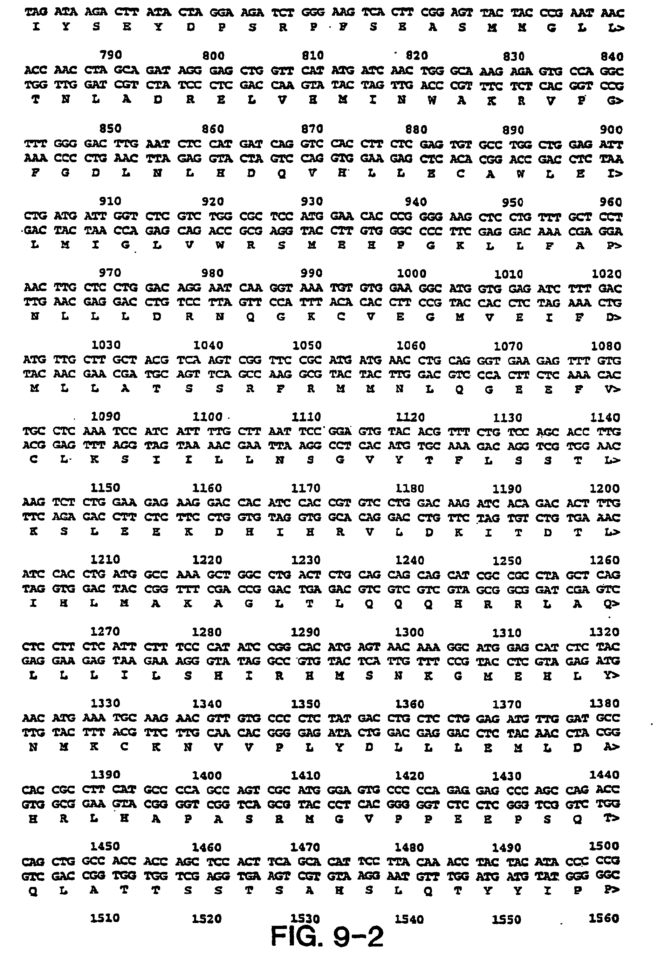

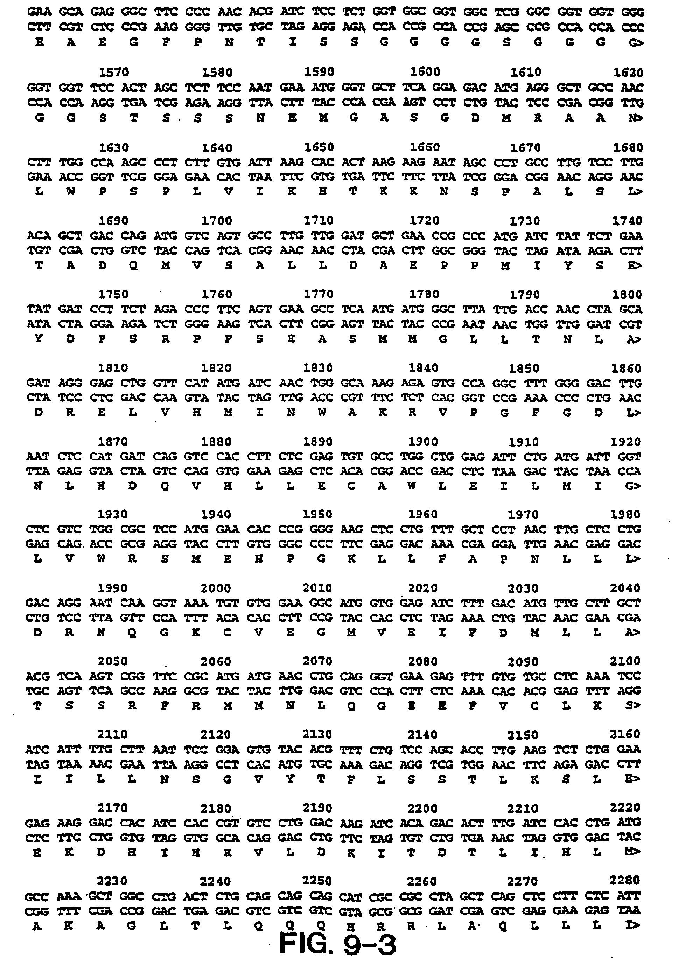

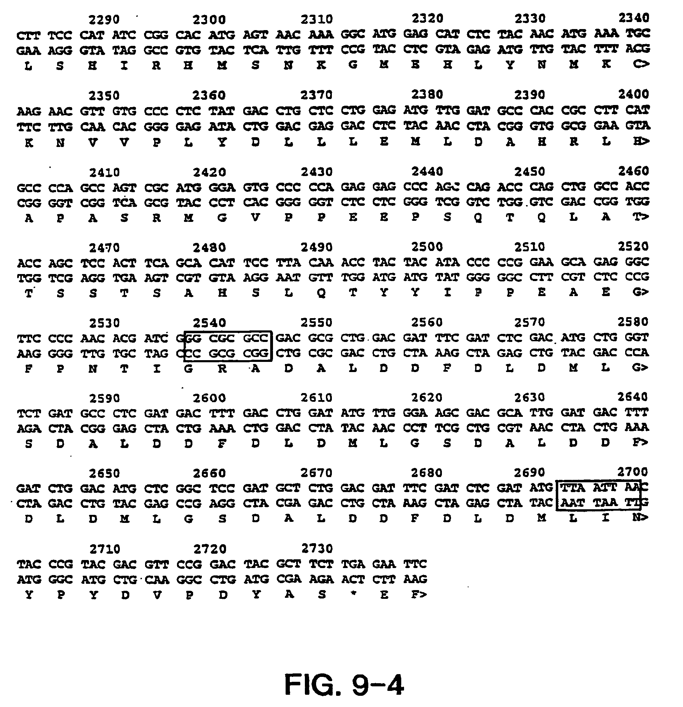

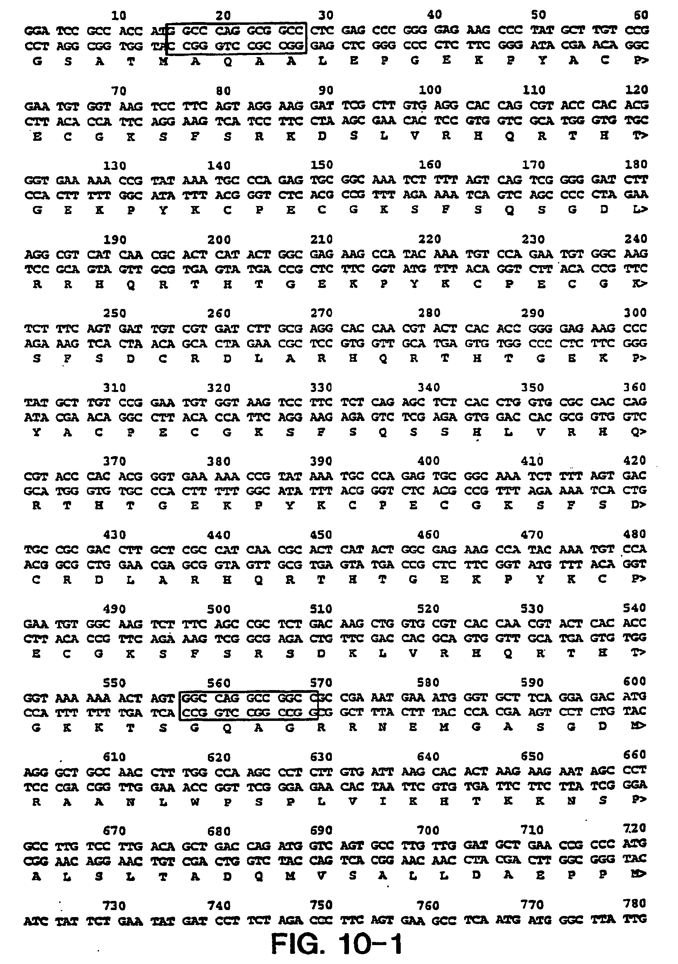

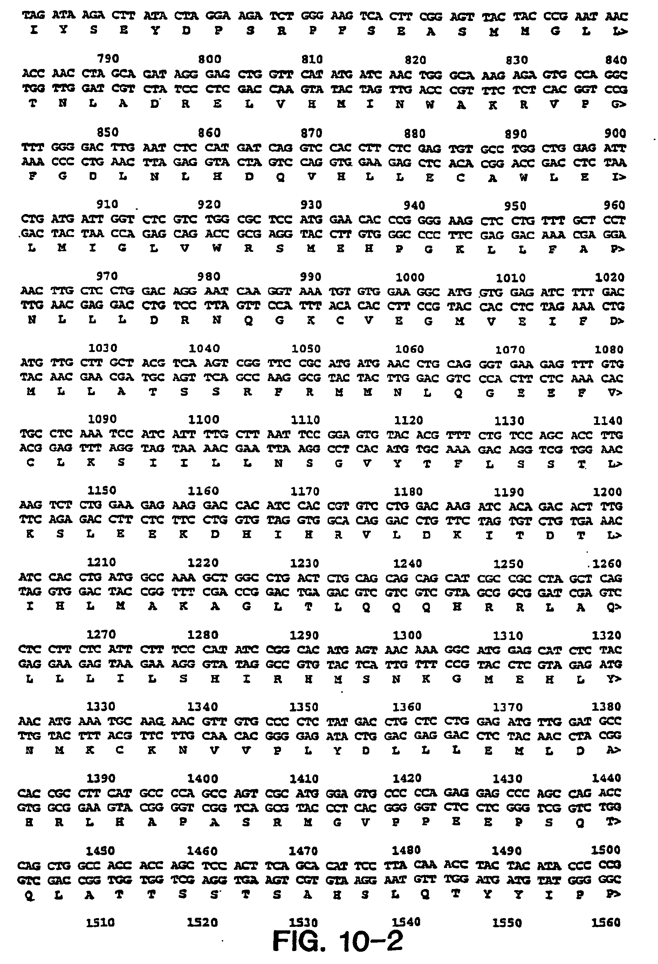

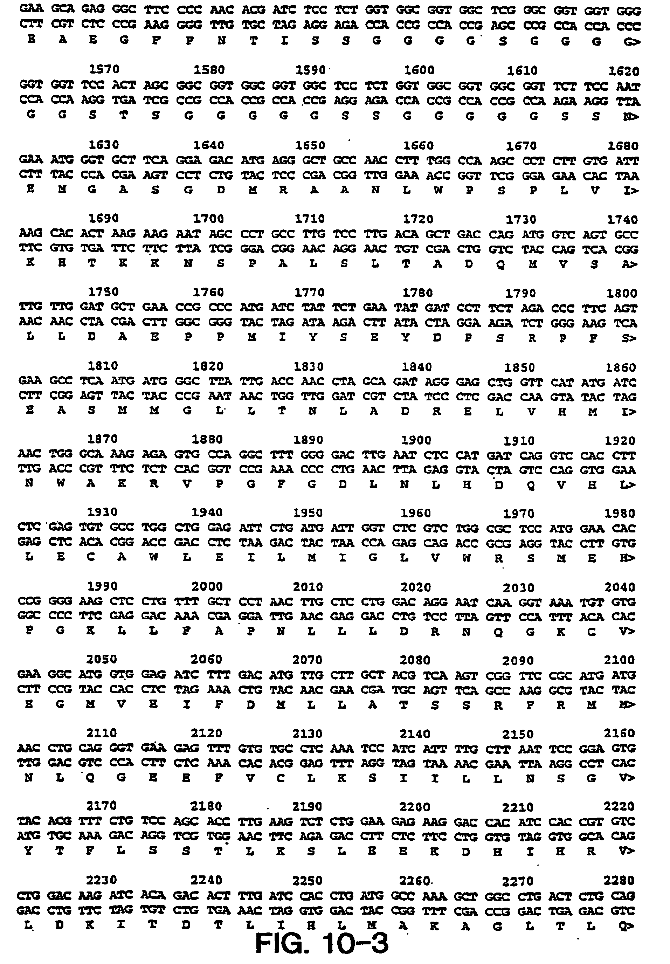

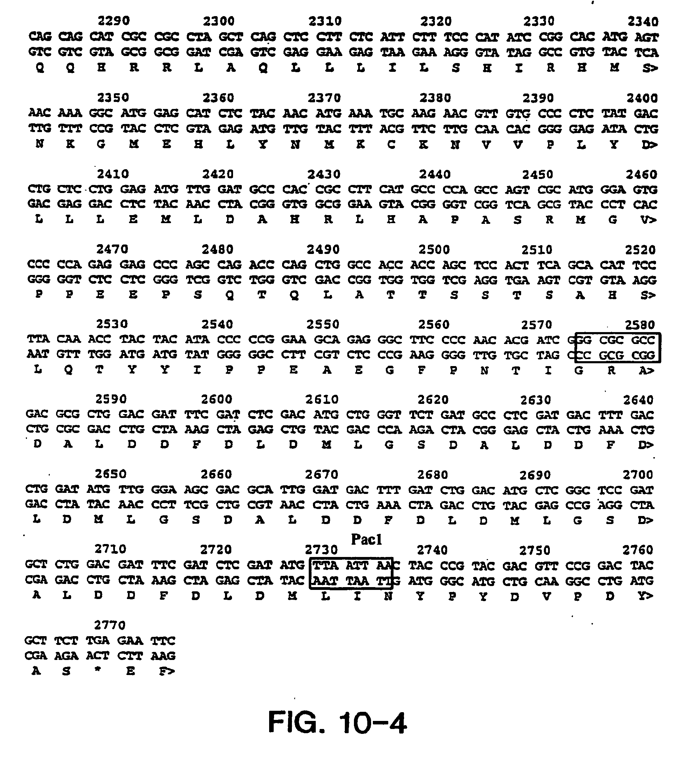

The nucleotide (SEQ ID NO: 39) and amino acid residue sequence (SEQ ID NO: 40) of polypeptide E2C-ER-L-ER-VP64 are shown in FIG. 9. The nucleotide (SEQ ID NO: 41) and amino acid residue sequence (SEQ ID NO: 42) of polypeptide E2C-ER-LL-ER-VP64 are shown in Fig. 10.

### III. Polynucleotides, Expression Vectors and Host Cells

In a related aspect, the present invention provides polynucleotides that encode a polypeptide gene switch of this invention, expression vectors containing those polynucleotides, cells containing those polynucleotides and transformed cells containing those expression vectors. Vectors of primary utility for gene therapy include, but are not limited to human adenovirus vectors, adeno-associated vectors, murine or lenti virus derived retroviral vectors, or a variety of non-viral compositions including liposomes, polymers, and other DNA containing conjugates. Such vector systems can be used o deliver the gene switches either *in vitro* or *in vivo*, depending on the vector system. With adenovirus, for instance, vectors can be administered intravenously to transduce the liver and other organs, introduced directly into the lung, or into vascular compartments temporarily localized by ligation or other methods. Methods for constructing such vectors, and methods and uses for the described invention are known to those skilled in the field of gene therapy.

### IV. Methods of Regulating Nucleotide Function

The present invention further provides a process for regulating the expression of a desired nucleotide sequence such as a gene. In accordance with the process, the target nucleotide sequence is exposed to an effective amount of a gene switch and a ligand, wherein the nucleotide binding domain of the gene switch binds to a portion of the target nucleotide and wherein the ligand binds to at least one of the ligand binding domains of the gene switch. Exposure can occur *in vitro* or *ex vivo.* The term "effective amount" means that amount that regulates transcription of a nucleotide (e.g. structural gene or translation of RNA).

The term "regulating" refers to the suppression, enhancement, or induction of a function. For example, a polypeptide of the invention may modulate a promoter sequence by binding to a motif within the promoter, thereby enhancing or suppressing transcription of a gene operatively linked to the promoter nucleotide sequence. Alternatively, modulation may include inhibition of a gene where the polypeptide binds to the structural gene and blocks DNA dependent RNA polymerase from reading through the gene, thus inhibiting transcription of the gene. Alternatively, modulation may include inhibition of translation of a transcript.

The promoter region of a gene includes the regulatory elements that typically lie 5' to a structural gene. If a gene is to be activated, proteins known as transcription factors attach to the promoter region of the gene. This assembly resembles an "on switch" by enabling an enzyme to transcribe a second genetic segment from DNA to RNA. In most cases the resulting RNA molecule serves as a template for synthesis of a specific protein; sometimes RNA itself is the final product.

Regulation of gene expression or transcription can be accomplished both by exposing the target geneto a polypeptide switch of this invention or, preferably by transforming in vitro or ex vivo a cell that contains the target gene with an expression vector that contains a polynucleotide sequence that encodes a gene switch.

The Examples that follow illustrate particular embodiments of the present invention and are not limiting of the specification or claims in any way.

### EXAMPLE 1: General Methods

**Construction of zinc finger proteins**. For the construction of the B3 and N1 zinc finger proteins, DNA recognition helices from the Zif268 Finger 2 variants pmGAA, pmGAC, pmGGA, pmGGG, and pGTA were utilized [Segal, D. J., Dreier, B., Beerli, R. R., and Barbas, C. F., III (1999) Proc. Natl. Acad. Sci. USA 96, 2758-2763]. Three finger proteins binding the respective 9-bp target-sites were constructed by grafting the appropriate DNA recognition helices into the framework of the three finger protein Sp1C [Desjarlais, J. R., and Berg, J. M. (1993) Proc. Natl Acad. Sci. USA 90, 2256-2260]; DNA fragments encoding the two 3 finger proteins were assembled from 6 overlapping oligonucleotides as described [Beerli, R. R., Segal, D. J., Dreier, B., and Barbas, C. F., III (1998) Proc. Natl. Acad. Sci. USA 95, 14628-14633]. The three finger protein coding regions were then cloned into the bacterial expression vector pMal-CSS via Sfi1 digestion.

**Protein purification.** Moltose binding protein (MBP) fusion proteins were purified to >90% homogeneity using the Protein Fusion and Purification System (New England Biolabs), except that Zinc Buffer A (ZBA; 10 mM Tris, pH7.5/90 mM KCI, 1 mM MgCl₂, 90 µM ZnCl₂)/1% BSA /5 mM DTT) was used as the column buffer. Protein purity and concentration were determined from Coomassie blue-stained 15% SDS-PAGE gels by comparison to BSA standards.

**ELISA analysis**. In 96-well ELISA plates, 0.2 µg of streptavidin (Pierce) was applied to each well for 1 hour at 37°C, then washed twice with water. Biotinylated target oligonucleotide (0.025 µg) was applied in the same manner. ZBA/3% BSA was applied for blocking, but the wells were not washed after incubation. All subsequent incubations were at room temperature. Starting with 2 µg purified MBP fusion protein in the top wells, 2-fold serial dilutions were applied in 1x binding buffer (ZBA/1 % BSA/5 mM DIT/0.12 µg/µl sheared herring sperm DNA). The samples were incubated 1 hour at room temperature, followed by 10 washes with water. Mouse anti-maltose binding protein mAb (Sigma) in ZBA/1% BSA was applied to the wells for 30 minutes, followed by 10 washes with water. Goat anti -mouse IgG mAb conjugated to alkaline phosphatase (Sigma) was applied to the wells for 30 minutes, followed by 10 washes with water. Alkaline phosphatase substrate (Sigma) was applied, and the OD₄₀₅ was quantitated with SOFTmax 235 (Molecular Devices).

**Gel mobility shift assays**. Target oligonucleotides were labeled at their 3' ends with [³²P] and gel purified. Eleven 3-fold serial dilutions of protein were incubated in 20 µl binding reactions (1x Binding Buffer/10% glycerol/≈1 pM target oligonucleotide) for three hours at room temperature, then resolved on a 5% polyacrlyamide gel in 0.5x TBE buffer. Quantitation of dried gels was performed using a PhosphorImager and ImageQuant software (Molecular Dynamics), and the K_{D} was determined by Scatchard analysis.

**Reporter constructs for determining the optimal spacing and orientation of the two half-sites.** C7 dimer-TATA fragments were generated by PCR amplification with C7 dimer-TATA primers (5'-GAG GGT ACC GCG TGG GCG A₀₋₅ GCG TGG GCG AGT CGA CTC TAG AGG GTA TAT AAT GG-3' (SEQ ID NO: 1) for direct repeats; 5'-GAG GGT ACC GCG TGG GCG A₀₋₅ CGC CCA CGC AGT CGA CTC TAG AGG GTA TAT AAT GG-3' (SEQ ID NO: 2) for inverted repeats; 5'-GAG GGT ACC CGC CCA CGC A₀₋₅ GCG TGG GCG AGT CGA CTC TAG AGG GTA TAT AAT GG-3' (SEQ ID NO: 3) for everted repeats) and GLprimer2 (5'-CTT TAT GTT TTT GGC GTC TTC C-3' (SEQ ID NO: 4); Promega), using p17x4TATA-luc (gift from S. Y. Tsai) as a template. PCR products were cloned into pGL3-Basic (Promega) via digestion with the restriction endonucleases Kpnl and Nco1.

**RU486- and Tamoxifen-inducible promoter constructs**. 10xC7-TATA, 10xB3-TATA, and 10xN1-TATA fragments were assembled from two pairs of complementary oligonucleotides each and cloned into Sacl-Xmal linearized pGL3-Basic (Promega), upstream of the firefly luciferase coding region, creating the plasmids 10xC7-TATA-luc, 10xB3-TATA-luc, and 10xN1-TATA-luc. To generate the 10xN1-TATA-lacZ reporter construct, the lacZ coding region was excised from pβgal-Basic (Clontech) and used to replace the luciferase coding region of 10xN1-TATA-luc via Hind3-BamH1 digestion.

**Luciferase and β-gal reporter assays**. For all transfections, HeLa cells were plated in 24-well dishes and used at a confluency of 40-60%. For luciferase reporter assays, 175 ng reporter plasmid (promotor constructs in pGL3 or, as negative control, pGL3-Basic) and 25 ng effector plasmid (zinc finger-steroid receptor fusions in pcDNA3 or, as negative control, empty pcDNA3) were transfected using the Lipofectamine reagent (Gibco BRL). After approximately 24 h, expression was induced by the addition of 10nM RU486 (Biomol), 100 nM 4-OHT (Sigma), or 5 mM Ponasterone A (Invitrogen). Cell extracts were prepared approximately 48 hours after transfection and assayed for luciferase activity using the Promega luciferase assay reagent in a MicroLumat LB96P luminometer (EG&G Berthold, Gaithersburg, MD). For dual reporter assays, 85ng luciferase reporter plasmid, 85ng b-gal reporter plasmid, and 15ng of each of the two effector plasmids were transfected. b-gal activity was measured using the luminescent b-galactosidase detection kit II (Clontech).

**Zinc finger-steroid receptor fusion constructs with N-terminal effector domains.** The VP16 coding region was PCR amplified from pcDNA3/C7-VP16 using the primers VPNhe-F (5'-GAG GAG GAG GAG GCT AGC GCC ACC ATG GGG CGC GCC GGC GCT CCC CCG ACC GAT GTC AGC CTG-3') (SEQ ID NO: 5), and VPHind-B (5'-GAG GAG GAG GAG AAG CTT GTT AAT TAA ACC GTA CTC GTC AAT TCC AAG GGC ATC G-3') (SEQ ID NO: 6) or VPNLSHind-B (5'-GAG GAG GAG GAG AAG CTT AAC TTT GCG TTT CTT TTT CGG GTT AAT TAA ACC GTA CTC GTC AAT TCC AAG GGC ATC G-3') (SEQ ID NO: 7). The C7 coding region was amplified from the same plasmid, using the primers C7Hind-F (5'-GAG GAG GAG GAG AAG CTT GGG GCC ACG GCG GCC CTC GAG CCC TAT GC-3') (SEQ ID NO: 8), and C7Bam-B (5'-GAG GAG GGA TCC CCC TGG CCG GCC TGG CCA CTA GTT CTA GAG TC-3') (SEQ ID NO: 9) or C7NLSBam-B (5'-GAG GAG GGA TCC CCA ACT TTG CGT TTC TTT TTC GGC TGG CCG GCC TGG CCA CTA GTT CTA GAG TC-3') (SEQ ID NO: 10). The human PR truncated LBD (aa645-914) was amplified from PAPCMVGL914VPc'-SV [Wang, Y., Xu, J., Pierson, T., O'Malley, B. W., and Tsai, S. Y. (1997) Gene Therapy 4, 432-441] using the primers PRBam-F (5'-GAG GAG GAG GAG GGA TCC AGT CAG AGT TGT GAG AGC ACT GGA TGC TG-3') (SEQ ID NO: 11) and PREco-B (5'-GAG GAG GAA TTC TCA AGC AAT AAC TTC AGA CAT CAT TTC TGG AAA TTC-3') (SEQ ID NO: 12). The VP16-C7-PR, VP16-NLS-C7-PR, and VP16-C7-NLS-PR coding regions were then assembled in pcDNA3.1(+)Zeo (Invitrogen) using the Nhe1, Hind3, BamH1, and EcoR1 restriction sites incorporated in the PCR primers. In the resulting constructs, the C7 coding regions were flanked by two Sfi1 sites, and the VP16 coding regions by Asc1 and Pac1 sites. These restriction sites were introduced to facilitate the exchange of DBDs and effector domains, respectively.

To generate the VP16-C7-ER, VP16-NLS-C7-ER, and VP16-C7-NLS-ER constructs, the point-mutated murine ER LBD coding region (aa281-599, G525R) was excised from pBabe/Myc-ER [Littlewood, T. D., Hancock, D. C., Danielian, P. S., Parker, M. G., and Evan, G. L (1995) Nucl. Acids Res. 23, 1686-1690], and used to replace the PR LBD coding region via BamHl-EcoRl restriction digestion.

To generate fusion constructs with B3 or N1 DBDs, C7 was replaced by the B3 or N1 coding regions via Sfi1 digestion. Fusion constructs containing a VP64 effector domain were produced by replacing VP16 by the VP64 coding region via Asc1-Pac1 digestion.

**Zinc finger-steroid receptor fusion constructs with C-terminal effector domains.** The truncated human PR LBD was amplified from PAPCMVGL914VPc'-SV [Wang, Y., Xu, J., Pierson, T., O'Malley, B. W., and Tsai, S. Y. (1997) Gene Therapy 4, 432-441] using the primers PRFse-F (5'- GAG GAG GAG GAG GAG GGC CGG CCG CGT CGA CCA GGT CAG AGT TGT GAG AGC ACT GGA TGC-3) (SEQ ID NO: 13) and PRAsc-B (5'- GAG GAG GAG GAG GAG GGC GCG CCC CGT CGA CCC AGC AAT AAC TTC AGA CAT CAT TTC TGG-3') (SEQ ID NO: 14). The point-mutated mouse ER LBD was amplified from pBabe/Myc-ER [Littlewood, T. D., Hancock, D. C., Danielian, P. S., Parker, M. G., and Evan, G. I. (1995) Nucl. Acids Res. 23, 1686-1690] using the primers ERFse-F (5'- GAG GAG GAG GAG GAG GGC CGG CCG CCG AAA TGA AAT GGG TGC TTC AGG AGA C-3') (SEQ ID NO: 15) and ERAsc-B (5'- GAG GAG GAG GAG GAG GGC GCG CCC GAT CGT GTT GGG GAA GCC CTC TGC TTC-3') (SEQ ID NO: 16). The resulting PCR products were then inserted into pcDNA3/E2C-VP16 [Beerli, R. R., Segal, D. J., Dreier, B., and Barbas, C. F., III (1998) Proc. Natl. Acad. Sci. USA 95, 14628-14633], in between the E2C and VP16 coding regions, via digestion with the restriction endonucleases Fse1 and Asc1.

To generate fusion constructs with B3 or N1 DBDs, E2C was replaced by the B3 or N1 coding regions via Sfi1 digestion. Fusion constructs containing a VP64 effector domain were produced by replacing VP16 by the VP64 coding region via Asc1-Pac1 digestion.

**Heterodimeric switch constructs**. For construction of the E2C-ER fusion, the point-mutated mouse ER LBD was amplified from pBabe/Myc-ER [Littlewood,.T. D., Hancock, D. C., Danielian, P. S., Parker, M. G., and Evan, G. I. (1995) Nucl. Acids Res. 23, 1686-1690] using the primers ERFse-F and ERPac-B (5'-GAG GAG GAG GAG GAG TTA ATT AAG ATC GTG TTG GGG AAG CCC TCT GCT TC-3') (SEQ ID NO: 17). The PCR product was then inserted into the construct pcDNA3/E2C-VP64, replacing the VP64 coding region, via Fse1-Pac1 digestion. To generate the ER-VP64 fusion, the ER LBD was amplified using the primers ERATGBam-F (5'-GAG GAG GAG GAG GGA TCC GCC ACC ATG CGA AAT GAA ATG GGT GCT TCA GGA GAC-3') (SEQ ID NO: 18) and ERAsc-B. The PCR product was then inserted into pcDNA3/E2C-VP64, [Beerli, R. R., Segal, D. J., Dreier, B., and Barbas, C. F., III (1998).Proc. Natl. Acad. Sci. USA 95, 14628-14633] replacing the E2C coding region, via BamH1-Asc1 digestion.

**Single-chain switch constructs**. For construction of single-chain fusions with two ER LBDs, the point-mutated mouse ER LBD was amplified from pBabe/Myc-ER [Littlewood, T. D., Hancock, D. C., Danielian, P. S., Parker, M. G., and Evan, G. I. (1995) Nucl. Acids Res. 23, 1686-1690] either using the primers ERFse-F and ERSpe1-B (5'-GAG GAG GAG GAG GAG GAG ACT AGT GGA ACC ACC CCC ACC ACC GCC CGA GCC ACC GCC ACC AGA GGA GAT CGT GTT GGG GAA GCC CTC TGC-3') (SEQ ID NO: 19), or using the primers ERNhe1-F1 (for 18aa linker construct; 5'-GAG GAG GAG GAG GAG GAG GCT AGC GGC GGT GGC GGT GGC TCC TCT GGT GGC GGT GGC GGT TCT TCC AAT GAA ATG GGT GCT TCA GGA GAC-3') (SEQ ID NO: 20) or ERNhel-F2 (for 30aa linker construct; 5'- GAG GAG GAG GAG GAG GAG GCT AGC TCT TCC AAT GAA ATG GGT GCT TCA GGA GAC -3') (SEQ ID NO: 21), and ERAsc-B. The PCR products were then digested with, respectively, Fse1 and Spe1, or Nhe1 and Asc1, and inserted into Fse1-Asc1 linearized pcDNA3/E2C-VP64 [Beerli, R. R., Segal, D. J., Dreier, B., and Barbas, C. F., III (1998) Proc. Natl. Acad. Sci. USA 95, 14628-14633].

For construction ofRXR-EcR single-chain fusions, the ligand binding domain of the human retinoid X receptor (hRXRα, aa373-654) was PCR amplified from pVgRXR (Invitrogen) using the primers RXRFse-F (5'-GAG GAG GAG GGC CGG CCG GGA AGC CGT GCA GGA GGA GCG GC-3') (SEQ ID NO: 22) and RXRSpe-B (5'-GAG GAG GAG GAG GAG ACT AGT GGA ACC ACC CCC ACC ACC GCC CGA GCC ACC GCC ACC AGA GGA AGT CAT TTG GTG CGG CGC CTC CAG C-3') (SEQ ID NO: 23). The ligand binding domain of the ecdysone receptor (EcR, aa202-462, *drosophila melanogaster)* was PCR amplified from pVgRXR using the primers EcRNhe-F1 (for 18aa linker construct; 5'-GAG GAG GAG GAG GCT AGC TCT TCC GGT GGC GGC CAA GAC TTT GTT AAG AAG G-3') (SEQ ID NO: 24), or EcRNhe-F2 (for 30aa linker construct; 5'-GAG GAG GAG GAG GCT AGC GGC GGT GGC GGT GGC TCC TCT GGT GGC GGT GGC GGT TCT TCC GGT GGC GGC CAA GAC TTT GTT AAG AAG G-3') (SEQ ID NO: 25), and EcRAsc-B (5'-GAG GAG GAG GGC GCG CCC GGC ATG AAC GTC CCA GAT CTC CTC GAG-3') (SEQ ID NO: 26). The PCR products were then digested with, respectively, Fse1 and Spe1, or Nhe1 and Asc1, and inserted into Fse1-Asc1 linearized pcDNA3/E2C-VP64 [Beerli, R. R., Segal, D. J., Dreier, B., and Barbas, C. F., III (1998) Proc. Natl. Acad. Sci. USA 95, 14628-14633]. DNA binding domains were exchanged via Sfi1 digestion, effector domains via Asc1-Pac1 digestion.

To generate the 36aa linker, E2C-RLLE-VP64 fusion construct, the RXR LBD was PCR amplified from pcDNA3/E2C-RE-VP64 using the primers RXRFse-F and RXRSpeLL-B (5'-GAG GAG GAG GAG GAG ACT AGT AGA GCC ACC GCC CCC TTC AGA ACC GCC CGA GCC ACC GCC ACC AGA GG-3') (SEQ ID NO: 27). The EcR LBD was amplified from the same plasmid, using the primers EcRNheLL-F (5'-GAG GAG GAG GAG GCT AGC GGG GGT TCG GAG GGT GGC GGG TCT GAG GGT GGG GGT GGT TCC ACT AGC TCT TCC-3') (SEQ ID NO: 28) and EcRAsc-B. The PCR products were inserted into pcDNA3/E2C-VP64 as described above.

### EXAMPLE 2: Gene Switches

**Generation of hormone-regulated zinc finger-steroid receptor fusion proteins.** Previous studies have shown the potential of engineered C2-H2 zinc finger proteins for the regulation of target gene expression [. Liu, Q., Segal, D. J., Ghiara, J. B., and Barbas, C. F., III (1997) Proc. Natl. Acad. Sci. USA 94, 5525-5530; Kim, J. S., and Pabo, C. O. (1997) J Biol Chem 272,29795-29800; Beerli, R. R, Segal, D. J., Dreier, B., and Barbas, C. F., III (1998) Proc. Natl. Acad. Sci. USA 95, 14628-14633; Beerli, R. R., Dreier, B., and Barbas, C. F., III (2000) Proc. Natl. Acad. Sci. USA 97, 1495-1500]. However, to fully realize the potential of engineered zinc finger proteins, it is desirable that their otherwise constitutive DNA binding activity be rendered ligand-dependent. The ligand binding domains (LBDs) of the human progesterone receptor (hPR) and the murine estrogen receptor (mER) have previously been used for the regulation of heterologous proteins, after having been modified to lack binding to the natural hormones while retaining binding to synthetic antagonists [Littlewood, T. D., Hancock, D. C., Danielian, P. S., Parker, M. G., and Evan, G. I. (1995) Nucl. Acids Res. 23,1686-1690; Wang, Y., Xu, J., Pierson, T., O'Malley, B. W., and Tsai, S. Y. (1997) Gene Therapy 4, 432-441]. Thus, the Zif268 variant C7 [Wu, H., Yang, W.-P., and Barbas, C. F., III (1995) Proc. Natl. Acad. Sci. USA 92, 344-348] was fused to a transcriptional activation domain plus the LBD of either of the two nuclear hormone receptors. The VP64-C7-PR fusion protein contains an N-terminal VP64 activation domain [Beerli, R. R., Segal, D. J., Dreier, B., and Barbas, C. F., III (1998) Proc. Natl. Acad. Sci. USA 95, 14628-14633], and a C-terminal hPR LBD (aa645-914) lacking amino acids 915-933, responsive to the progesterone-antagonist RU486/Mifepristone but not to progesterone [Wang, Y., Xu, J., Pierson, T., O'Malley, B. W., and Tsai, S. Y. (1997) Gene Therapy 4, 432-441]. The VP64-C7-ER fusion protein contains a C-terminal mER LBD (aa282-599) with a single amino acid substitution (G525R), and is responsive to the estrogen antagonist 4-hydroxy-tamoxifen (4-OHT) but not to estrogen [Littlewood, T. D., Hancock, D. C., Danielian, P. S., Parker, M. G., and Evan, G. I. (1995) Nucl. Acids Res. 23, 1686-1690].

**Determination of the optimal response element for zinc finger-steroid receptor fusion proteins.** Naturally occurring steroid receptors bind DNA as dimers and typically recognize response elements consisting of palindromic sequences [Evans, R. M. (1988) Science 240, 889-895; Carson-Jurica, M. A., Schrader, W. T., and O'Malley, W. (1990) Endocrine Reviews 11, 201-220]. Moreover, it was demonstrated that in some cases also direct repeats can serve as binding sites for receptor dimers [Aumais, J. P., Lee, H. S., DeGannes, C., Horsford, J., and White, J. H. (1996) J. Biol. Chem. 271, 12568-12577]. Given this obvious flexibility in DNA recognition by naturally occurring receptor dimers, the optimal structure of a response element for an artificial, zinc finger based transcriptional switch was not known. However, to develop an efficient, hormone-inducible system for the regulation of target gene expression, a detailed knowledge of the binding site architecture is required.

To determine the optimal orientation and spacing of the two half-sites of a response element for a zinc finger-LBD fusion protein, a series of reporter plasmids was constructed. Each contains two C7 binding sites upstream of a TATA box and a firefly luciferase coding region. The two C7 binding sites were introduced in different orientations (direct, inverted, or everted repeat) and with various spacings (no spacing or 1 to 5 bp spacing). Plasmids directing expression of VP64-C-PR or VP64-C7-ER fusion constructs were then co-transfected with the various reporter plasmids and assayed for hormone-induced luciferase expression. Significantly, each of the C7 dimer binding sites was able to act as a response element for both PR and ER based proteins, albeit at variing efficiency. In contrast, a reporter plasmid with a single C7 binding site was not activated, indicating that hormone-induced activation of transcription was mediated by dimers.

Optimal spacing depended on the orientation of the two half-sites. In the case of the PR fusion protein, optimal spacing seemed to be at 2-3 bp for inverted repeats and 3 bp for everted repeats. Response elements consisting of direct repeats had no single optimal spacing; the best response was obtained with 4-5 bp, or no spacing at all. For the ER fusion protein, optimal spacing was at 3-4 bp for direct repeats, 1-2 bp for inverted repeats, and 3 bp for everted repeats. It should be noted that there were significant variations in the basal, i.e. ligand-independent activity of PR and ER fusion proteins, depending on the response element tested. Most notably, increasing the spacing of direct repeats from 3 to 4 bp led to a 1.9-fold higher basal activity of VP64-C7-PR, and even a 3.7-fold increase in the case of VP64-C7-ER. High basal activity is extremely undesirable for an inducible promoter system, where tight control over the expression levels of a particular gene of interest is often required, especially if the gene product is toxic. Thus, in choosing appropriate response elements, particular attention must be paid not only to hormone inducibility but also to its basal activity in the presence of the regulatory protein. The response element consisting of direct repeats with a spacing of three nucleotides was considered to be a good choice for use in a hormone-inducible artificial promoter, since it was compatible with both PR and ER fusion proteins. Significantly, its basal acticity in the presence of either PR or ER fusion proteins was among the lowest of all response elements tested. Furthermore, good hormone induced activation of transcription was observed with both VP64-C7-PR (3.9-fold) and VP64-C7-ER (9.5-fold).

**Generation of novel DNA binding domains**. While the use of the C7 DNA binding domain was well suited for the preliminary studies described above, it may not be a good choice for incorporation into an inducible transcriptional regulator. The C7 protein is a variant of the mouse transcription factor Zif268 [Pavletich, N. P., and Pabo, C. O. (1991) Science 252, 809-817], with increased affinity but unchanged specificity [Wu, H., Yang, W.-P., and Barbas, C. F., III (1995) Proc. Natl. Acad. Sci. USA 92, 344-348]. We reasoned that the use of alternate DNA binding domains would minimize potential pleiotropic effects of the chimeric regulators. Previously, we described a strategy for the rapid assembly of zinc finger proteins from a family of predefined zinc finger domains specific for each of the sixteen 5'-GNN-3' DNA triplets [Beerli, R. R., Segal, D. J., Dreier, B., and Barbas, C. F., III (1998) Proc. Natl Acad. Sci. USA 95,14628-14633; Segal, D. J., Dreier, B., Beerli, R. R., and Barbas, C. F., III (1999) Proc. Natl. Acad. Sci. USA 96, 2758-2763]. Three finger proteins binding any desired 5'-(GNN)₃-3'sequence can be rapidly prepared by grafting the amino acid residues involved in base-specific DNA recognition into the framework of the consensus three finger protein Sp1C [Desjarlais, J. R., and Berg, J. M. (1993) Proc. Natl. Acad. Sci. USA 90, 2256-2260]. To date, well over 100 three finger proteins have been produced in our laboratory. Two of these, B3 and N1, were chosen to be used in inducible transcriptional regulators (Figure 1A). The B3 and N1 proteins are designed to bind the sequences 5'-GGA GGG GAC-3' or 5'-GGG GTA GAA-3', respectively. To verify their DNA binding specificity, these proteins were purified as MBP-fusions and tested by ELISA analysis using an arbitrary selection of oligonucleotides containing 5'-(GNN)₃-3' sequences (Fig. 1B). Significantly, both proteins recognized their target sequence and showed no crossreactivity to any of the other 5'-(GNN)₃-3'sequences tested. However, as judged by ELISA, binding of N1 was much weaker than binding of B3. Therefore, affinities were determined by electrophoretic mobility-shift analysis. The B3 protein bound its target sequence with a K_{D} value of 15nM, similar to the K_{D} values we previously reported for other three finger proteins [Beerli, R. R., Segal, D. J., Dreier, B., and Barbas, C. F., III (1998) Proc. Natl. Acad. Sci. USA 95, 14628-14633]. In contrast, N1 affinity for its target was dramatically lower and we estimate its K_{D} value to be in the range of 5-10 µM. The fact that the two proteins had very different affinities for their respective target sequences was considered positive, since it allows to investigate the influence of affinity on the functionality of an inducible expression system.

**RU486- and 4-OHT-inducible systems for the control of gene expression.** To allow for a comparative analysis, a series of RU486- or 4-OHT-inducible transcriptional regulators were constructed containing either the B3 or the N1 DNA binding domain. The role of placement of the activation domain was investigated, by fusing it either to the N- or the C-terminus of the protein. Two different activation domains were compared: the *herpes simplex* virus VP16 transactivation domain [Sadowski, I., Ma, J., Triezenberg, S., and Ptashne, M. (1988) Nature 335, 563-564], and the synthetic VP64 activation domain, which consists of 4 tandem repeats of VP16's minimal activation domain [Beerli, R. R., Segal, D. J., Dreier, B., and Barbas, C. F., III (1998) Proc. Natl. Acad. Sci. USA 95, 14628-14633].

Synthetic promoters were constructed based on the B3 and N1 DNA target sequences, and the optimal response element structure defined above. The 10xB3-TATA-luc and 10xN1-TATA-luc plasmids each contain five response elements, consisting of direct repeats spaced by three nucleotides, upstream of a TATA box and a firefly luciferase coding region. The response elements are separated from each other by six nucleotides, which should allow the concomitant binding of five dimers and thus maximize the promoter activity. The activity of the various fusion constructs was assessed by transient cotransfection studies with the cognate TATA reporter plasmids in HeLa cells (Table 1).

**Table 1**

| | LBD=PR | | LBD=ER | |
|---|---|---|---|---|
| | exp.1 | exp.2 | exp.1 | exp.2 |
| VP16-B3-LBD | 34x | 36x | 37x | 26x |
| VP64-B3-LBD | 37x | 24x | 26x | 27x |
| B3-LBD-VP16 | 115x | 116x | 47x | 58x |
| B3-LBD-VP64 | 110x | 85x | 62x | 99x |
| VP16-N1-LBD | 188x | 159x | 101x | 39x |
| VP64-N1-LBD | 206x | 390x | 49x | 58x |
| N1-LBD-VP16 | 282x | 203x | 24x | 30x |
| N1-LBD-VP64 | 151x | 129x | 1319x | 464x |

In general, the ER fusion proteins turned out to be the stronger transactivators, and 4-OHT-induced luciferase activity was usually 3 to 6 times higher than RU486-induced luciferase activity mediated by PR fusion proteins. However, since the basal, *i.e.* ligand independent, activity of ER chimeras was often somewhat higher, their hormone-induced fold-stimulation was not generally better. Hormone-dependent gene activation in excess of 2 orders of magnitude was commonly observed with both PR and ER fusion proteins, values that are significantly better than what was previously reported for the Gal4-PR fusion protein GLVPc' [Wang, Y., Xu, J., Pierson, T., O'Malley, B. W., and Tsai, S. Y. (1997) Gene Therapy 4, 432-441].

The placement of the activation domain had a significant influence on the activity of the chimeric regulators. However, favored placement was dependent on the nature of the activation domain. Whereas the VP16 domain yielded the more potent activators when placed at the C-terminus, the VP64 was more active at the N-terminus. Accordingly, direct comparisons showed that an N-terminal VP64 was more potent than a N-terminal VP16 domain, and a C-terminal VP16 was more potent than a C-terminal VP64 domain. The nature and placement of the activation domain was also found to have an influence on the basal activity of the chimeric regulators. In particular, a relatively high basal activity was observed in the case of regulators with N-terminal VP64 domain.

The nature of the DNA binding domain had a major influence on the extent of ligand-dependence of the chimeras. Use of the N1 protein as DNA binding domain led to more tightly regulated fusion constructs with significantly better fold-stimulation of promoter activities than the use of B3, likely due to the dramatic affinity differences between N1 and B3. In particular, the N1-ER-VP64 regulator had no significant basal activity and was capable of mediating a 464- to 1319-fold 4-OHT-induced activation of the lOxNI-TATA minimal promoter (Table 1). The extent of ligand-induced activation of gene expression over a range of 3 orders of magnitude is particularly remarkable, since it has thus far only been reported for the tetracycline controlled system of gene regulation [Gossen, M., and Bujard, H. (1992) Proc. Natl. Acad. Sci. USA 89, 5547-5551; Gossen, M., Freundlieb, S., Bender, G., Müller, G., Hillen, W., and Bujard, H.(1995) Science 268,1766-1769].

**Concomitant regulation of multiple promoters.** Zinc finger technology has made a large repertoire of DNA binding specificities available for use in protein engineering [Beerli, R. R., Segal, D. J., Dreier, B., and Barbas, C. F., III (1998) Proc. Natl. Acad. Sci. USA 95, 14628-1463; Segal, D. J., Dreier, B., Beerli, R. R., and Barbas, C. F., III (1999) Proc. Natl. Acad. Sci. USA 96,2758-2763; Beerli, R. R., Dreier, B., and Barbas, C. F., III (2000) Proc. Natl. Acad. Sci. USA 97, 1495-1500]. The availability of different steroid hormone receptor-derived regulatory domains [Littlewood, T. D., Hancock, D. C., Danielian, P. S., Parker, M. G., and Evan, G. I. (1995) Nucl. Acids Res. 23,1686-1690; Wang, Y., Xu, J., Pierson, T., O'Malley, B. W., and Tsai, S. Y. (1997) Gene Therapy 4,432-441], and the ability to redirect chimeric regulators to virtually any desired target sequence should make it possible to independently regulate the expression of multiple genes at the same time. To examine this possibility, a reporter plasmid was constructed directing expression of β-galactosidase (β-gal) under the control of the 10xN1-TATA minimal promoter. The chimeric regulators B3-PR-VP16 and N1-ER-VP64 were then transiently expressed in HeLa cells along with the 10xB3-TATA-1uc and 10xN1-TATA-β-gal reporter plasmids. The transfected cells were treated with either RU486 or 4-OHT and the luciferase and β-gal activities were monitored. Significantly, RU486 induced expression of luciferase while having no effect on β-gal reporter gene activity. 4-OHT, on the other hand, did not affect luciferase expression but efficiently activated β-gal expression. These results demonstrate that the two regulator/promoter combinations act independently from one another, and that multiple genes can efficiently and independently regulated by the selective addition of the desired hormone.

**Development of a monomeric hormone-dependent gene-switch. The** ability to engineer DNA binding proteins with desired specificities makes it possible to generate artificial transcription factors capable of imposing dominant regulatory effects on endogenous genes [Beerli, R R., Dreier, B., and Barbas, C. F., III (2000) Proc. Natl. Acad. Sci. USA 97, 1495-1500]. For many applications of this technology it may be desirable that the effect on endogenous gene expression is reversible. The use of steroid hormone receptor LBDs has the potential to render regulation of endogenous gene expression reversible. However, one major drawback is the fact that steroid hormone receptors, as well as the chimeric regulators described herein, bind DNA as dimers. Thus, when the fusion protein C7-ER-VP64 was transiently expressed in HeLa cells it was unable to regulate a reporter construct carrying a single C7 binding site, while it readily regulated a reporter that had two C7 binding sites and therefore accommodated binding of a dimer (Fig. 2B). An additional problem was encountered when the C7 DBD was replaced by E2C, which contains six zinc finger domains and recognizes the 18-bp sequence 5'-GGG GCC GGA GCC GCA GTG-3' (SEQ ID NO; 29) in the 5'-UTR of the proto-oncogene *c-erbB-2* [Yamamoto, T., Ikawa, S., Akiyama, T., Semba, K., Nomura, N., Miyajima, N., Saito, T., and Toyoshima, K. (1986) Nature 319, 230-234; Beerli, R. R., Segal, D. J., Dreier, B., and Barbas, C. F., III (1998) Proc. Natl. Acad. Sci. USA 95, 14628-14633]. The E2C-ER-VP64 fusion protein was constitutively active on a reporter carrying a single E2C binding site, almost as active as an E2C-VP64 fusion without an ER LBD, and did not respond well to hormone. Apparently, the use of a large DNA binding domain recognizing an extended stretch of DNA with high affinity renders the chimera hormone- and dimerization-independent.

To overcome these problems, we produced two types of ER-based chimeric regulators, designed to be capable of regulating gene expression through a single binding site in a hormone-dependent manner. In the first strategy, a heterodimeric regulator was generated consisting of the engineered zinc finger protein E2C fused to an ER LBD, as well as an ER LBD fused to a VP64 activation domain (Fig. 2A). When this heterodimeric regulator was expressed in HeLa cells, it had no significant activity on the E2C-TATA-luc reporter plasmid in the absence of 4-OHT. Addition of hormone led to a 3- to 5-fold stimulation of luciferase expression, indicating the formation of functional heterodimers. However, hormone-induced reporter gene activation was significantly lower than that induced by an E2C-VP64 fusion protein, presumably at least in part due to the formation of E2C-ER and ER-VP64 homodimers. Homodimers were inactive, since neither E2C-ER nor ER-VP64 alone induced luciferase expression. In the second strategy, fusion proteins were generated by combining the dimerization partners E2C-ER and ER-VP64 in one single polypeptide, through a flexible polypeptide linker. Two linkers were tested, 18 and 30 amino acids in length, creating the proteins E2C-scER/18-VP64 and E2C-scER/30-VP64 (Fig. 2A). These proteins were expected to be activated via intramolecular, rather than intermolecular, dimerization and therefore functional as monomers. Combination of two ER LBDs into one single-chain fusion construct should allow a more efficient hormone-induced dimerization and therefore yield more efficient activators. Indeed, when E2C-scER/18-VP64 and E2C-scER/30-VP64 were transiently expressed in HeLa cells, they efficiently activated the E2C-TATA-luc reporter in a largely hormone-dependent manner (Fig. 2B, 2C and 2D). Thus, dimeric regulators requiring response elements similar to those of natural steroid hormone receptors were successfully converted into monomeric, ligand-dependent transcription factors.

**Monomeric gene-switch based on EcR and RXR LBDs.** To show that the production of a ligand-dependent monomeric gene switch by fusion with two LBDs is a generally applicable strategy, the utility of other nuclear hormone receptors was tested. In particular, utility of the LBDs of the *Drosophila* ecdysone receptor (EcR) was investigated. In *Drosophila,* this receptor functions as a heterodimer between EcR and the product of the *ultraspiracle* (USP) gene [Yao, T.-P., Forman, B. M., Jiang, Z., Cherbas, L., Chen, J.-D., McKeown, M., Cherbas, P., and Evans, R. M. (1993) Nature 366, 476-479]. However, it has been shown that EcR also efficiently heterodimerizes with USP's vertebrate homologue retinoid X receptor (RXR) in response to the ecdysone agonists Muristerone A or Ponasterone A (PonA) [Nakanishi, K. (1992) Steroids 57, 649-657; Yao, T.-P., Forman, B. M., Jiang, Z., Cherbas, L., Chen, J.-D., McKeown, M., Cherbas, P., and Evans, R M. (1993) Nature 366, 476-479; No, D., Yao, T.-P., and Evans, R. M. (1996) Proc. Natl. Acad. Sci. USA 93,3346-3351]. The EcR and RXR LBDs were therefore used to prepare a monomeric gene switch analogous to the scER chimeras described above (Fig. 3A). Thus, the human RXRα LBD (aa373-654) and the *Drosophila* EcR LBD (aa202-462) were inserted in between the E2C DBD and the VP64 activation domain, creating E2C-RE-VP64. In this fusion construct, the two LBDs are connected by an 18 amino acid flexible linker, the same that was used in E2C-scER/18-VP64. When this chimeric regulator was transiently expressed in HeLa cells along with the E2C-TATA-luc reporter plasmid, significant basal activity was observed. However, activity could be increased 3-fold by PonA, showing that this artificial construct was hormone-responsive. To improve the ligand dependence, the length of the linker connecting the RXR and EcR LBDs was increased, a measure that seemed beneficial in the case of the single-chain ER constructs. A longer linker should allow the LBDs to optimize their contact and add to the conformational disorder in the unliganded state. Indeed, when the linker was elongated to 30 aa (in E2C-RLE-VP64) or 36 aa (in E2C-RLLE-VP64), basal activity was significantly reduced and PonA led to a 9- to 10-fold activation, an extent of responsiveness comparable to the one of the single-chain ER fusion constructs (Fig. 3B). Thus, serial connection of pairs of nuclear hormone receptor LBDs appears to be a generally applicable strategy to render monomeric DNA binding proteins ligand-dependent.

The hPR and mER LBDs used for the fusion proteins did not encompass their natural SV40-like nuclear localization signals (NLS), located between amino acids 637 and 644 in hPR, and between amino acids 260 and 267 in mER [Carson-Jurica, M. A., Schrader, W. T., and O'Malley, W. (1990) Endocrine Reviews 11, 201-220]. While it has been shown that this NLS is not required for hormone-dependent nuclear localization of hPR, regulation of the subcellular localization of steroid receptors appears to be complex, and it was not *a priori* clear whether the presence of the SV40-like NLS was required for proper function of the chimeric proteins. Thus, additional constructs were prepared that incorporated an SV40 NLS (PKKKRKV) (SEQ ID NO: 30) in single letter amino acid code), either between VP16 and C7, or between C7 and LBD.

The chimeric transcriptional regulators were then tested for their ability to regulate the 10xC7-TATA-luc reporter plasmid in a hormone dependent manner. 10xC7-TATA-luc contains ten C7 binding sites [5'-GCG TGG GCG-3] spaced by 5 nucleotides, and a TATA box, upstream of the firefly luciferase coding region. Each of the fusion proteins upregulated expression of luciferase in a largely hormone dependent manner. RU486 stimulated the activity of VP16-C7 PR 26-fold, while 4-OHT led to a 43-fold activation of VP16-C7-ER. There was no detectable crossreactivity between RU486 and ER, or between 4-OHT and PR. The presence of a NLS in either position was not only not required, but even undesirable, since it led to an increased basal (i.e. hormone-independent) activity of the fusion constructs, presumably through increased nuclear localization. Thus, the hPR (aa645-914) and mER (aa281-599, G525R) LBDs are able to confer hormone-dependence onto the zinc finger protein C7.

The ability to reversibly control the expression of multiple genes, or alleles of a gene, could prove very useful for many basic research applications. In particular, selective and independent expression of one gene, but not another (and *vice versa*), by small and nontoxic ligands would allow for a comparative analysis of gene function, both *in vitro* and in vivo. We have shown that our modular system for controlling target gene expression is indeed able to independently control the expression of two genes within the same transfected cell, as evidenced by RU486-dependent luciferase induction and 4-OHT-induced β-gal expression. The lack of β-gal induction by RU486, and luciferase induction by 4-OHT convincingly demonstrates the specificity of the chimeric regulators described here. Not only is the exquisite specificity of the utilized DNA binding domains retained, but also there is no detectable crossreaction between RU486 and the ER LBD, or between 4-OHT and the PR LBD.

## Claims

1. A non-naturally occurring polypeptide comprising two ligand binding domains derived from nuclear hormone receptors operatively linked to a DNA binding domain and a transcriptional regulation domain, wherein the DNA binding domain comprises at least one engineered zinc finger binding motif.

2. The polypeptide of claim 1 wherein the two ligand binding domains are covalently linked by means of a peptide linker.

3. The polypeptide of claim 2 wherein the linker contains from about 10 to about 40 amino acid residues.

4. The polypeptide of claim 2 wherein the linker contains from about 15 to about 35 amino acid residues.

5. The polypeptide of claim 2 wherein the linker contains from about 18 to about 30 amino acid residues.

6. The polypeptide of claim 1 wherein the first and second ligand binding domains are derived from different nuclear hormone receptors.

7. The polypeptide of claim 1 wherein the first and second ligand binding domains are derived from the same nuclear hormone receptor.

8. The polypeptide of claim 1 wherein the nuclear hormone receptor is an estrogen receptor, a progesterone receptor, a retinoic acid receptor, or a retinoid X receptor.

9. The polypeptide of claim 6 wherein at least one of the ligand binding domains is derived from a retinoid X receptor.

10. The polypeptide of claim 1 that comprises from two to twelve engineered - zinc finger DNA binding motifs.

11. The polypeptide of claim 1 that comprises from two to six engineered zinc finger binding motifs.

12. The polypeptide of claim 10 wherein the engineered zinc finger DNA binding motifs specifically bind to a nucleotide sequence of the formula (GNN)₁₋₆, where G is guanine and N is any nucleotide.

13. The polypeptide of claim 1 wherein the transcriptional regulating domain is an activation domain.

14. The polypeptide of claim 1 wherein the transcriptional regulating domain is a repression domain.

15. A non-naturally occurring polypeptide comprising: (a) a DNA binding domain having from two to six engineered zinc finger DNA binding motfis; (b) a first ligand binding domain derived from a retinoid X receptor operatively linked to the DNA binding domain, a second ligand binding domain derived from an ecdysone receptor operatively linked to the first ligand binding domain with a peptide spacer of from 18 to 36 amino acid recidues; and (c) a transcriptional regulating domain operatively linked to the second ligand binding domain.

16. A non-naturally occurring polypeptide comprising: (a) a DNA binding domain having from two to six engineered zinc finger DNA binding motifs; (b) a first ligand binding domain derived from a progesterone receptor operatively linked to the DNA binding domain, a second ligand binding domain derived from a progesterone receptor linked to the frist ligand binding domain with a peptide spacer of from 18 to 36 amino acid residues; and (c) a transcriptional regulating domain operatively linked to the second ligand binding domain.

17. A polynucleotide that encodes the polypeptide of claim 1.

18. A polynucleotide that encodes the polypeptide of any of claims 15 or 16.

19. An expression vector comprising the polynucleotide of claim 17.

20. An expression vector comprising the polynucleotide of claim 18.

21. A host cell comprising the polynucleotide of any of claims 17 or 18.

22. A host cell comprising the expression vector of any of claims 19 or 20.

23. A process of regulating the function of a target nucleotide that contains a defined sequence, the process comprising exposing in vitro or ex vivo the target nucleotide to the polypeptide of claim 1 in the presence of a ligand that binds one of the ligand binding domains of the polypeptide, wherein the DNA binding domain of the polypeptide binds the defined sequence.

24. A process according to claim 23 wherein said ligand is a non-native ligand.

25. An expression vector encoding a non-naturally occurring polypeptide according to any of claim 1 to 16 for use in gene therapy.

## Patentansprüche

1. Nicht natürlich vorkommendes Polypeptid, umfassend zwei von nukleären Hormonrezeptoren stammende Ligandenbindungsdomänen, die funktionsfähig mit einer DNA-Bindungsdomäne verknüpft sind, und eine Transkriptionsregulationsdomäne, wobei die DNA-Bindungsdomäne wenigstens ein manipuliertes Zinkfinger-Bindungsmotiv umfasst.

2. Polypeptid nach Anspruch 1, wobei die zwei Ligandenbindungsdomänen mittels eines Peptidlinkers kovalent miteinander verknüpft sind.

3. Polypeptid nach Anspruch 2, wobei der Linker etwa 10 bis etwa 40 Aminosäurereste enthält.

4. Polypeptid nach Anspruch 2, wobei der Linker etwa 15 bis etwa 35 Aminosäurereste enthält.

5. Polypeptid nach Anspruch 2, wobei der Linker etwa 18 bis etwa 30 Aminosäurereste enthält.

6. Polypeptid nach Anspruch 1, wobei die erste und zweite Ligandenbindungsdomäne von unterschiedlichen nukleären Hormonrezeptoren stammen.

7. Polypeptid nach Anspruch 1, wobei die erste und zweite Ligandenbindungsdomäne vom gleichen nukleären Hormonrezeptor stammen.

8. Polypeptid nach Anspruch 1, wobei der nukleäre Hormonrezeptor ein Estrogenrezeptor, ein Progesteronrezeptor, ein Retinsäurerezeptor oder ein Retinoid-X-Rezeptor ist.

9. Polypeptid nach Anspruch 6, wobei wenigstens eine der Ligandenbindungsdomänen von einem Retinoid-X-Rezeptor stammt.

10. Polypeptid nach Anspruch 1, das zwei bis zwölf manipulierte Zinkfinger-DNA-Bindungsmotive umfasst.

11. Polypeptid nach Anspruch 1, das zwei bis sechs manipulierte Zinkfinger-Bindungsmotive umfasst.

12. Polypeptid nach Anspruch 10, wobei die manipulierten Zinkfinger-DNA-Bindungsmotive spezifisch an eine Nukleotidsequenz der Formel (GNN)₁₋₆ binden, wobei G Guanin ist und N ein beliebiges Nukleotid ist.

13. Polypeptid nach Anspruch 1, wobei die Transkriptionsregulationsdomäne eine Aktivierungsdomäne ist.

14. Polypeptid nach Anspruch 1, wobei die Transkriptionsregulationsdomäne eine Repressionsdomäne ist.

15. Nicht natürlich vorkommendes Polypeptid umfassend: (a) eine DNA-Bindungsdomäne mit zwei bis sechs manipulierten Zinkfinger-DNA-Bindungsmotiven; (b) eine erste von einem Retinoid-X-Rezeptor stammende Ligandenbindungsdomäne, die funktionsfähig mit der DNA-Bindungsdomäne verknüpft ist, eine zweite von einem Ecdysonrezeptor stammende Ligandenbindungsdomäne, die mit einem Peptidspacer aus 18 bis 36 Aminosäureresten funktionsfähig mit der ersten Ligandenbindungsdomäne verknüpft ist; und (c) eine Transkriptionsregulationsdomäne, die funktionsfähig mit der zweiten Ligandenbindungsdomäne verknüpft ist.

16. Nicht natürlich vorkommendes Polypeptid, umfassend: (a) eine DNA-Bindungsdomäne mit zwei bis sechs manipulierten Zinkfinger-DNA-Bindungsmotiven; (b) eine erste von einem Progesteronrezeptor stammende Ligandenbindungsdomäne, die funktionsfähig mit der DNA-Bindungsdomäne verknüpft ist, eine zweite von einem Progesteronrezeptor stammende Ligandenbindungsdomäne, die mit einem Peptidspacer aus 18 bis 36 Aminosäureresten mit der ersten Ligandenbindungsdomäne verknüpft ist; und (c) eine Transkriptionsregulationsdomäne, die funktionsfähig mit der zweiten Ligandenbindungsdomäne verknüpft ist.

17. Polynukleotid, das für das Polypeptid nach Anspruch 1 codiert.

18. Polynukleotid, das für das Polypeptid nach irgendeinem der Ansprüche 15 oder 16 codiert.

19. Expressionsvektor, umfassend das Polynukleotid nach Anspruch 17.

20. Expressionsvektor, umfassend das Polynukleotid nach Anspruch 18.

21. Wirtszelle, umfassend das Polynukleotid nach irgendeinem der Ansprüche 17 oder 18.

22. Wirtszelle, umfassend den Expressionsvektor nach irgendeinem der Ansprüche 19 oder 20.

23. Verfahren zum Regulieren der Funktion eines Zielnukleotids, das eine definierte Sequenz enthält, wobei das Verfahren die *in vitro-* oder *ex vivo*-Exposition des Zielnukleotids mit dem Polypeptid nach Anspruch 1 in Gegenwart eines Liganden umfasst, der eine der Ligandenbindungsdomänen des Polypeptids bindet, wobei die DNA-Bindungsdomäne des Polypeptids die definierte Sequenz bindet.

24. Verfahren gemäß Anspruch 23, wobei der Ligand ein nicht-nativer Ligand ist.

25. Expressionsvektor, der für ein nicht natürlich vorkommendes Polypeptid gemäß irgendeinem der Ansprüche 1 bis 16 codiert, zur Gentherapie.

## Revendications

1. Polypeptide non naturel comprenant deux domaines de liaison à un ligand dérivant de récepteurs hormonaux nucléaires liés de manière opérationnelle à un domaine de liaison à l'ADN et à un domaine de régulation de la transcription, dans lequel le domaine de liaison à l'ADN comprend au moins un motif de liaison en doigt de zinc spécifiquement conçu.

2. Polypeptide selon la revendication 1, dans lequel les deux domaines de liaison à un ligand sont liés de manière covalente au moyen d'un lieur peptidique.

3. Polypeptide selon la revendication 2, dans lequel le lieur contient d'environ 10 à environ 40 résidus d'acides aminés.

4. Polypeptide selon la revendication 2, dans lequel le lieur contient d'environ 15 à environ 35 résidus d'acides aminés.

5. Polypeptide selon la revendication 2, dans lequel le lieur contient d'environ 18 à environ 30 résidus d'acides aminés.

6. Polypeptide selon la revendication 1, dans lequel les premier et deuxième domaines de liaison à un ligand dérivent de récepteurs hormonaux nucléaires différents.

7. Polypeptide selon la revendication 1, dans lequel les premier et deuxième domaines de liaison à un ligand dérivent du même récepteur hormonal nucléaire.

8. Polypeptide selon la revendication 1, dans lequel le récepteur hormonal nucléaire est un récepteur d'oestrogène, un récepteur de progestérone, un récepteur d'acide rétinoïque, ou un récepteur de rétinoïde X.

9. Polypeptide selon la revendication 6, dans lequel au moins l'un des domaines de liaison à un ligand dérive d'un récepteur de rétinoïde X.

10. Polypeptide selon la revendication 1, qui comprend de deux à douze motifs de liaison à l'ADN en doigt de zinc spécifiquement conçus.

11. Polypeptide selon la revendication 1, qui comprend de deux à six motifs de liaison en doigt de zinc spécifiquement conçus.

12. Polypeptide selon la revendication 10, dans lequel les motifs de liaison à l'ADN en doigt de zinc spécifiquement conçus se lient de manière spécifique à une séquence de nucléotides de formule (GNN)₁₋₆, où G est la guanine et N est un nucléotide quelconque.

13. Polypeptide selon la revendication 1, dans lequel le domaine de régulation de la transcription est un domaine d'activation.

14. Polypeptide selon la revendication 1, dans lequel le domaine de régulation de la transcription est un domaine de répression.

15. Polypeptide non naturel comprenant : (a) un domaine de liaison à l'ADN ayant de deux à six motifs de liaison à l'ADN en doigt de zinc spécifiquement conçus ; (b) un premier domaine de liaison à un ligand dérivant d'un récepteur de rétinoïde X lié de manière opérationnelle au domaine de liaison à l'ADN, un deuxième domaine de liaison à un ligand dérivant d'un récepteur d'ecdysone lié de manière opérationnelle au premier domaine de liaison à un ligand avec un écarteur peptidique de 18 à 36 résidus d'acides aminés ; et (c) un domaine de régulation de la transcription lié de manière opérationnelle au deuxième domaine de liaison à un ligand.

16. Polypeptide non naturel comprenant : (a) un domaine de liaison à l'ADN ayant de deux à six motifs de liaison à l'ADN en doigt de zinc spécifiquement conçus ; (b) un premier domaine de liaison à un ligand dérivant d'un récepteur de progestérone lié de manière opérationnelle au domaine de liaison à l'ADN, un deuxième domaine de liaison à un ligand dérivant d'un récepteur de progestérone lié de manière opérationnelle au premier domaine de liaison à un ligand avec un écarteur peptidique de 18 à 36 résidus d'acides aminés ; et (c) un domaine de régulation de la transcription lié de manière opérationnelle au deuxième domaine de liaison à un ligand.

17. Polynucléotide qui code le polypeptide de la revendication 1.

18. Polynucléotide qui code le polypeptide de l'une quelconque des revendications 15 ou 16.

19. Vecteur d'expression comprenant le polynucléotide de la revendication 17.

20. Vecteur d'expression comprenant le polynucléotide de la revendication 18.

21. Cellule hôte comprenant le polynucléotide de l'une quelconque des revendications 17 ou 18.

22. Cellule hôte comprenant le vecteur d'expression de l'une quelconque des revendications 19 ou 20.

23. Procédé pour réguler la fonction d'un nucléotide cible qui contient une séquence définie, le procédé comprenant l'exposition *in vitro* ou *ex vivo* du nucléotide cible au polypeptide de la revendication 1 en présence d'un ligand qui se lie à l'un des domaines de liaison à un ligand du polypeptide, dans lequel le domaine de liaison à l'ADN du polypeptide se lie à la séquence définie.

24. Procédé selon la revendication 23, dans lequel ledit ligand est un ligand non natif.

25. Vecteur d'expression codant un polypeptide non naturel selon l'une quelconque des revendications 1 à 16, pour une utilisation en thérapie génique.


### SEQUENCE LISTING

<110> Novartis AG
   Novartis Erfindungen Verwaltungsgesellschaft mbH
   The Scripps Research Institute
   Barbas, Carlos F.
   Beerli, Roger
   Schopfer, Ulrich
<120> REGULATION OF GENE EXPRESSION USING
   SINGLE-CHAIN, MONOMERIC, LIGAND DEPENDENT POLYPEPTIDE SWITCHES
<130> 4-31549A/SCR
<160> 53
<170> FastSEQ for windows version 4.0
<210> 1
   <211> 58
   <212> DNA
   <213> unknown
<220>
   <223> synthesized
   <221> misc_feature
   <222> 19-23
   <223> Some or all may be missing
<400> 1
   gagggtaccg cgtgggcgaa aaagcgtggg cgagtcgact ctagagggta tataatgg 58
<210> 2
   <211> 58
   <212> DNA
   <213> unknown
<220>
   <223> synthesized
   <221> misc_feature
   <222> 19-23
   <223> Some or all may be missing
<400> 2
   gagggtaccg cgtgggcgaa aaacgcccac gcagtcgact ctagagggta tataatgg 58
<210> 3
   <211> 58
   <212> DNA
   <213> unknown
<220>
   <223> synthesized
   <221> misc_feature
   <222> 19-23
   <223> some or all may be missing
<400> 3
   gagggtaccc gcccacgcaa aaagcgtggg cgagtcgact ctagagggta tataatgg 58
<210> 4
   <211> 22
   <212> DNA
   <213> unknown
<220>
   <223> synthesized
<400> 4
   ctttatgttt ttggcgtctt cc 22
<210> 5
   <211> 63
   <212> DNA
   <213> Unknown
<220>
   <223> synthesized
<400> 5
   gaggaggagg aggctagcgc caccatgggg cgcgccggcg ctcccccgac cgatgtcagc 60
   ctg 63
<210> 6
   <211> 55
   <212> DNA
   <213> Unknown
<220>
   <223> synthesized
<400> 6
   gaggaggagg agaagcttgt taattaaacc gtactcgtca attccaaggg catcg 55
<210> 7
   <211> 76
   <212> DNA
   <213> unknown
<220>
   <223> synthesized
<400> 7
   gaggaggagg agaagcttaa ctttgcgttt ctttttcggg ttaattaaac cgtactcgtc 60
   aattccaagg gcatcg 76
<210> 8
   <211> 47
   <212> DNA
   <213> unknown
<220>
   <223> Synthesized
<400> 8
   gaggaggagg agaagcttgg ggccacggcg gccctcgagc cctatgc 47
<210> 9
   <211> 44
   <212> DNA
   <213> unknown
<220>
   <223> synthesized
<400> 9
   gaggagggat ccccctggcc ggcctggcca ctagttctag agtc 44
<210> 10
   <211> 65
   <212> DNA
   <213> unknown
<220>
   <223> synthesized
<400> 10
   gaggagggat ccccaacttt gcgtttcttt ttcggctggc cggcctggcc actagttcta 60
   gagtc 65
<210> 11
   <211> 47
   <212> DNA
   <213> unknown
<220>
   <223> synthesized
<400> 11
   gaggaggagg agggatccag tcagagttgt gagagcactg gatgctg 47
<210> 12
   <211> 48
   <212> DNA
   <213> Unknown
<220>
   <223> Synthesized
<400> 12
   gaggaggaat tctcaagcaa taacttcaga catcatttct ggaaattc 48
<210> 13
   <211> 60
   <212> DNA
   <213> unknown
<220>
   <223> synthesized
<400> 13
   gaggaggagg aggagggccg gccgcgtcga ccaggtcaga gttgtgagag cactggatgc 60
<210> 14
   <211> 60
   <212> DNA
   <213> unknown
<220>
   <223> Synthesized
<400> 14
   gaggaggagg aggagggcgc gccccgtcga cccagcaata acttcagaca tcatttctgg 60
<210> 15
   <211> 52
   <212> DNA
   <213> Unknown
<220>
   <223> Synthesized
<400> 15
   gaggaggagg aggagggccg gccgccgaaa tgaaatgggt gcttcaggag ac 52
<210> 16
   <211> 51
   <212> DNA
   <213> unknown
<220>
   <223> Synthesized
<400> 16
   gaggaggagg aggagggcgc gcccgatcgt gttggggaag ccctctgctt c 51
<210> 17
   <211> 50
   <212> DNA
   <213> Unknown
<220>
   <223> synthesized
<400> 17
   gaggaggagg aggagttaat taagatcgtg ttggggaagc cctctgcttc 50
<210> 18
   <211> 54
   <212> DNA
   <213> Unknown
<220>
   <223> synthesized
<400> 18
   gaggaggagg agggatccgc caccatgcga aatgaaatgg gtgcttcagg agac 54
<210> 19
   <211> 90
   <212> DNA
   <213> unknown
<220>
   <223> synthesized
<400> 19
   gaggaggagg aggaggagac tagtggaacc acccccacca ccgcccgagc caccgccacc 60
   agaggagatc gtgttgggga agccctctgc 90
<210> 20
   <211> 90
   <212> DNA
   <213> Unknown
<220>
   <223> synthesized
<400> 20
   gaggaggagg aggaggaggc tagcggcggt ggcggtggct cctctggtgg cggtggcggt 60
   tcttccaatg aaatgggtgc ttcaggagac 90
<210> 21
   <211> 54
   <212> DNA
   <213> unknown
<220>
   <223> synthesized
<400> 21
   gaggaggagg aggaggaggc tagctcttcc aatgaaatgg gtgcttcagg agac 54
<210> 22
   <211> 41
   <212> DNA
   <213> unknown
<220>
   <223> synthesized
<400> 22
   gaggaggagg gccggccggg aagccgtgca ggaggagcgg c 41
<210> 23
   <211> 88
   <212> DNA
   <213> Unknown
<220>
   <223> Synthesized
<400> 23
   gaggaggagg aggagactag tggaaccacc cccaccaccg cccgagccac cgccaccaga 60
   ggaagtcatt tggtgcggcg cctccagc 88
<210> 24
   <211> 52
   <212> DNA
   <213> unknown
<220>
   <223> synthesized
<400> 24
   gaggaggagg aggctagctc ttccggtggc ggccaagact ttgttaagaa gg 52
<210> 25
   <211> 88
   <212> DNA
   <213> unknown
<220>
   <223> synthesized
<400> 25
   gaggaggagg aggctagcgg cggtggcggt ggctcctctg gtggcggtgg cggttcttcc 60
   ggtggcggcc aagactttgt taagaagg 88
<210> 26
   <211> 45
   <212> DNA
   <213> Unknown
<220>
   <223> synthesized
<400> 26
   gaggaggagg gcgcgcccgg catgaacgtc ccagatctcc tcgag 45
<210> 27
   <211> 68
   <212> DNA
   <213> Unknown
<220>
   <223> synthesized
<400> 27
   gaggaggagg aggagactag tagagccacc gcccccttca gaaccgcccg agccaccgcc 60
   accagagg 68
<210> 28
   <211> 72
   <212> DNA
   <213> unknown
<220>
   <223> synthesized
<400> 28
   gaggaggagg aggctagcgg gggttcggag ggtggcgggt ctgagggtgg gggtggttcc 60
   actagctctt cc 72
<210> 29
   <211> 18
   <212> DNA
   <213> Unknown
<220>
   <223> synthesized
<400> 29
   ggggccggag ccgcagtg 18
<210> 30
   <211> 7
   <212> PRT
   <213> unknown
<220>
   <223> synthesized
<400> 30
   Pro Lys Lys Lys Arg Lys Val
   1 5
<210> 31
   <211> 558
   <212> DNA
   <213> unknown
<220>
   <223> synthesized; zinc finger binding domain B3B
   <221> misc_feature
   <222> 1, 2, 557, 558
   <223> n = A, T, C or G
<400> 31
   nnggcccagg cggccctcga gcccggggag aagccctatg cttgtccgga atgtggtaag 60
   tccttcagtc gcagcgatgt gctggtgcgc caccagcgta cccacacggg tgaaaaaccg 120
   tataaatgcc cagagtgcgg caaatctttt agccgcagcg atgatctggt tcgccatcaa 180
   cgcactcata ctggcgagaa gccatacaaa tgtccagaat gtggcaagtc tttctcccag 240
   tctagccacc tggttcgcca ccaacgtact cacaccgggg agaagcccta tgcttgtccg 300
   gaatgtggta agtccttcag ccgcagcgat aacctggtgc gccaccagcg tacccacacg 360
   ggtgaaaaac cgtataaatg cccagagtgc ggcaaatctt ttagccaggc cggccacctg 420
   gccagccatc aacgcactca tactggcgag aagccataca aatgtccaga atgtggcaag 480
   tctttcagtg attgtcgtga tcttgcgagg caccaacgta ctcacaccgg taaaaaaact 540
   agtggccagg ccggccnn 558
<210> 32
   <211> 186
   <212> PRT
   <213> unknown
<220>
   <223> synthesized; zinc finger binding domain B3B
<221> VARIANT
   <222> 1, 186
   <223> xaa = Any Amino Acid
<400> 32
   Xaa Ala Gln Ala Ala Leu Glu Pro Gly Glu Lys Pro Tyr Ala Cys Pro
   1               5                   10                  15
   Glu Cys Gly Lys Ser Phe Ser Arg Ser Asp Val Leu Val Arg His Gln
               20                  25                  30
   Arg Thr His Thr Gly Glu Lys Pro Tyr Lys Cys Pro Glu Cys Gly Lys
           35                  40                  45
   Ser Phe Ser Arg Ser Asp Asp Leu Val Arg His Gln Arg Thr His Thr
       50                  55                  60
   Gly Glu Lys Pro Tyr Lys Cys Pro Glu Cys Gly Lys Ser Phe Ser Gln
   65                  70                  75                  80
   Ser Ser His Leu Val Arg His Gln Arg Thr His Thr Gly Glu Lys Pro
                   85                  90                  95
   Tyr Ala Cys Pro Glu Cys Gly Lys Ser Phe Ser Arg Ser Asp Asn Leu
               100                 105                 110
   Val Arg His Gln Arg Thr His Thr Gly Glu Lys Pro Tyr Lys Cys Pro
           115                 120                 125
   Glu Cys Gly Lys Ser Phe Ser Gln Ala Gly His Leu Ala Ser His Gln
       130                 135                 140
   Arg Thr His Thr Gly Glu Lys Pro Tyr Lys Cys Pro Glu Cys Gly Lys
   145                 150                 155                 160
   Ser Phe Ser Asp Cys Arg Asp Leu Ala Arg His Gln Arg Thr His Thr
                   165                 170                 175
   Gly Lys Lys Thr Ser Gly Gln Ala Gly Xaa
               180                 185
<210> 33
   <211> 567
   <212> DNA
   <213> Unknown
<220>
   <223> synthesized; zinc finger binding domain 2c7
<221> misc_feature
   <222> 1, 2, 566, 567
   <223> n = A,T,C or G
<400> 33
   nnggcccagg cggccctcga gccctatgct tgccctgtcg agtcctgcga tcgccgcttt 60
   tctaagtcgg ctgatctgaa gcgccatatc cgcatccaca caggccagaa gcccttccag 120
   tgtcgaatat gcatgcgtaa cttcagtcgt agtgaccacc ttaccaccca catccgcacc 180
   cacacaggcg agaagccttt tgcctgtgac atttgtggga ggaagtttgc caggagtgat 240
   gaacgcaaga ggcataccaa aatccatacc ggtgagaagc cctatgcttg ccctgtcgag 300
   tcctgcgatc gccgcttttc taagtcggct gatctgaagc gccatatccg catccacaca 360
   ggccagaagc ccttccagtg tcgaatatgc atgcgtaact tcagtcgtag tgaccacctt 420
   accacccaca tccgcaccca cacaggcgag aagccttttg cctgtgacat ttgtgggagg 480
   aagtttgcca ggagtgatga acgcaagagg cataccaaaa tccatttaag acagaaggac 540
   tctagaacta gtggccaggc cggccnn                                     567
<210> 34
   <211> 189
   <212> PRT
   <213> unknown
<220>
   <223> Synthesized; zinc finger binding domain 2C7
   <221> VARIANT
   <222> 1, 189
   <223> Xaa = Any Amino Acid
<400> 34
   Xaa Ala Gln Ala Ala Leu Glu Pro Tyr Ala Cys Pro Val Glu Ser Cys
    1               5                  10                  15
   Asp Arg Arg Phe Ser Lys Ser Ala Asp Leu Lys Arg His Ile Arg Ile
               20                  25                  30
   His Thr Gly Gln Lys Pro Phe Gln Cys Arg Ile Cys Met Arg Asn Phe
           35                  40                  45
   Ser Arg Ser Asp His Leu Thr Thr His Ile Arg Thr His Thr Gly Glu
       50                  55                  60
   Lys Pro Phe Ala Cys Asp Ile Cys Gly Arg Lys Phe Ala Arg Ser Asp
   65                  70                  75                  80
   Glu Arg Lys Arg His Thr Lys Ile His Thr Gly Glu Lys Pro Tyr Ala
                   85                  90                  95
   Cys Pro Val Glu Ser Cys Asp Arg Arg Phe Ser Lys Ser Ala Asp Leu
               100                 105                 110
   Lys Arg His Ile Arg Ile His Thr Gly Gln Lys Pro Phe Gln Cys Arg
           115                 120                 125
   Ile Cys Met Arg Asn Phe Ser Arg Ser Asp His Leu Thr Thr His Ile
       130                 135                 140
   Arg Thr His Thr Gly Glu Lys Pro Phe Ala Cys Asp Ile Cys Gly Arg
   145                 150                 155                 160
   Lys Phe Ala Arg Ser Asp Glu Arg Lys Arg His Thr Lys Ile His Leu
                   165                 170                 175
   Arg Gln Lys Asp Ser Arg Thr Ser Gly Gln Ala Gly Xaa
               180                 185
<210> 35
   <211> 558
   <212> DNA
   <213> unknown
<220>
   <223> Synthesized; zinc finger binding domain B3C2
<221> misc_feature
   <222> 1, 2, 557, 558
   <223> n = A,T,C or G
<400> 35
   nnggcccagg cggccctcga gcccggggag aagccctatg cttgtccgga atgtggtaag 60
   tccttcagca ccagtggcca cctggtgcgc caccagcgta cccacacggg tgaaaaaccg 120
   tataaatgcc cagagtgcgg caaatctttt agtcgcagcg atgtgctggt gcgccatcaa 180
   cgcactcata ctggcgagaa gccatacaaa tgtccagaat gtggcaagtc tttctcacgt 240
   tcagacgact tggtccgtca ccaacgtact cacaccgggg agaagcccta tgcttgtccg 300
   gaatgtggta agtccttcag tgatcctggc aacctggttc gccaccagcg tacccacacg 360
   ggtgaaaaac cgtataaatg cccagagtgc ggcaaatctt ttagtcgctc cgataaactg 420
   gtgcgccatc aacgcactca tactggcgag aagccataca aatgtccaga atgtggcaag 480
   tctttctccc agtctagcca cctggttcgc caccaacgta ctcacaccgg taaaaaaact 540
   agtggccagg ccggccnn                                               558
<210> 36
   <211> 186
   <212> PRT
   <213> unknown
<220>
   <223> synthesized; zinc finger binding domain B3C2
<221> VARIANT
   <222> 1, 186
   <223> Xaa = Any Amino Acid
<400> 36
   Xaa Ala Gln Ala Ala Leu Glu Pro Gly Glu Lys Pro Tyr Ala Cys Pro
    1               5                  10                  15
   Glu Cys Gly Lys Ser Phe Ser Thr Ser Gly His Leu Val Arg His Gln
               20                  25                  30
   Arg Thr His Thr Gly Glu Lys Pro Tyr Lys Cys Pro Glu Cys Gly Lys
           35                  40                  45
   Ser Phe Ser Arg Ser Asp Val Leu Val Arg His Gln Arg Thr His Thr
       50                  55                  60
   Gly Glu Lys Pro Tyr Lys Cys Pro Glu Cys Gly Lys Ser Phe Ser Arg
   65                  70                  75                  80
   Ser Asp Asp Leu Val Arg His Gln Arg Thr His Thr Gly Glu Lys Pro
                   85                  90                  95
   Tyr Ala Cys Pro Glu Cys Gly Lys Ser Phe Ser Asp Pro Gly Asn Leu
               100                 105                 110
   Val Arg His Gln Arg Thr His Thr Gly Glu Lys Pro Tyr Lys Cys Pro
           115                 120                 125
   Glu Cys Gly Lys Ser Phe Ser Arg Ser Asp Lys Leu Val Arg His Gln
       130                 135                 140
   Arg Thr His Thr Gly Glu Lys Pro Tyr Lys Cys Pro Glu Cys Gly Lys
   145                 150                 155                 160
   Ser Phe Ser Gln Ser Ser His Leu Val Arg His Gln Arg Thr His Thr
                   165                 170                 175
   Gly Lys Lys Thr Ser Gly Gln Ala Gly Xaa
               180                 185
<210> 37
   <211> 303
   <212> DNA
   <213> unknown
<220>
   <223> synthesized; repression domain (KRAB-A)2
<400> 37
   gggcgcgccc aggaaactat gaccttcaaa gacgttgaag ttaccttctc tcaggacgaa 60
   tggggttggc tggactccgc tcagcgtaac ctgtaccgtg acgttatgct ggaaaactac 120
   cgcaacatgg cttccctggt tggcggcggc cgcggtggtc aggaaactat gaccttcaaa 180
   gacgttgaag ttaccttctc tcaggacgaa tggggttggc tggactccgc tcagcgtaac 240
   ctgtaccgtg acgttatgct ggaaaactac cgcaacatgg cttccctggt tggcttaatt 300
   aac                                                               303
<210> 38
   <211> 240
   <212> DNA
   <213> unknown
<220>
   <223> synthesized; repression domain (SID)2
<400> 38
   gggcgcgccg ctgccgtgcg catgaacatc cagatgctgc tcgaagccgc tgattatctg 60
   gaacgccggg agcgcgaagc cgagcacggc tacgccagca tgctgccata tggcggccgc 120
   ggtggtgccg ctgccgtgcg catgaacatc cagatgctgc tcgaagccgc tgattatctg 180
   gaacgccggg agcgcgaagc cgagcacggc tacgccagca tgctgccata tttaattaac 240
<210> 39
   <211> 2739
   <212> DNA
   <213> unknown
<220>
   <223> synthesized; polypeptide E2C-ER-L-ER-VP64
<400> 39
   ggatccgcca ccatggccca ggcggccctc gagcccgggg agaagcccta tgcttgtccg 60
   gaatgtggta agtccttcag taggaaggat tcgcttgtga ggcaccagcg tacccacacg 120
   ggtgaaaaac cgtataaatg cccagagtgc ggcaaatctt ttagtcagtc gggggatctt 180
   aggcgtcatc aacgcactca tactggcgag aagccataca aatgtccaga atgtggcaag 240
   tctttcagtg attgtcgtga tcttgcgagg caccaacgta ctcacaccgg ggagaagccc 300
   tatgcttgtc cggaatgtgg taagtccttc tctcagagct ctcacctggt gcgccaccag 360
   cgtacccaca cgggtgaaaa accgtataaa tgcccagagt gcggcaaatc ttttagtgac 420
   tgccgcgacc ttgctcgcca tcaacgcact catactggcg agaagccata caaatgtcca 480
   gaatgtggca agtctttcag ccgctctgac aagctggtgc gtcaccaacg tactcacacc 540
   ggtaaaaaaa ctagtggcca ggccggccgc cgaaatgaaa tgggtgcttc aggagacatg 600
   agggctgcca acctttggcc aagccctctt gtgattaagc acactaagaa gaatagccct 660
   gccttgtcct tgacagctga ccagatggtc agtgccttgt tggatgctga accgcccatg 720
   atctattctg aatatgatcc ttctagaccc ttcagtgaag cctcaatgat gggcttattg 780
   accaacctag cagataggga gctggttcat atgatcaact gggcaaagag agtgccaggc 840
   tttggggact tgaatctcca tgatcaggtc caccttctcg agtgtgcctg gctggagatt 900
   ctgatgattg gtctcgtctg gcgctccatg gaacacccgg ggaagctcct gtttgctcct 960
   aacttgctcc tggacaggaa tcaaggtaaa tgtgtggaag gcatggtgga gatctttgac 1020
   atgttgcttg ctacgtcaag tcggttccgc atgatgaacc tgcagggtga agagtttgtg 1080
   tgcctcaaat ccatcatttt gcttaattcc ggagtgtaca cgtttctgtc cagcaccttg 1140
   aagtctctgg aagagaagga ccacatccac cgtgtcctgg acaagatcac agacactttg 1200
   atccacctga tggccaaagc tggcctgact ctgcagcagc agcatcgccg cctagctcag 1260
   ctccttctca ttctttccca tatccggcac atgagtaaca aaggcatgga gcatctctac 1320
   aacatgaaat gcaagaacgt tgtgcccctc tatgacctgc tcctggagat gttggatgcc 1380
   caccgccttc atgccccagc cagtcgcatg ggagtgcccc cagaggagcc cagccagacc 1440
   cagctggcca ccaccagctc cacttcagca cattccttac aaacctacta catacccccg 1500
   gaagcagagg gcttccccaa cacgatctcc tctggtggcg gtggctcggg cggtggtggg 1560
   ggtggttcca ctagctcttc caatgaaatg ggtgcttcag gagacatgag ggctgccaac 1620
   ctttggccaa gccctcttgt gattaagcac actaagaaga atagccctgc cttgtccttg 1680
   acagctgacc agatggtcag tgccttgttg gatgctgaac cgcccatgat ctattctgaa 1740
   tatgatcctt ctagaccctt cagtgaagcc tcaatgatgg gcttattgac caacctagca 1800
   gatagggagc tggttcatat gatcaactgg gcaaagagag tgccaggctt tggggacttg 1860
   aatctccatg atcaggtcca ccttctcgag tgtgcctggc tggagattct gatgattggt 1920
   ctcgtctggc gctccatgga acacccgggg aagctcctgt ttgctcctaa cttgctcctg 1980
   gacaggaatc aaggtaaatg tgtggaaggc atggtggaga tctttgacat gttgcttgct 2040
   acgtcaagtc ggttccgcat gatgaacctg cagggtgaag agtttgtgtg cctcaaatcc 2100
   atcattttgc ttaattccgg agtgtacacg tttctgtcca gcaccttgaa gtctctggaa 2160
   gagaaggacc acatccaccg tgtcctggac aagatcacag acactttgat ccacctgatg 2220
   gccaaagctg gcctgactct gcagcagcag catcgccgcc tagctcagct ccttctcatt 2280
   ctttcccata tccggcacat gagtaacaaa ggcatggagc atctctacaa catgaaatgc 2340
   aagaacgttg tgcccctcta tgacctgctc ctggagatgt tggatgccca ccgccttcat 2400
   gccccagcca gtcgcatggg agtgccccca gaggagccca gccagaccca gctggccacc 2460
   accagctcca cttcagcaca ttccttacaa acctactaca tacccccgga agcagagggc 2520
   ttccccaaca cgatcgggcg cgccgacgcg ctggacgatt tcgatctcga catgctgggt 2580
   tctgatgccc tcgatgactt tgacctggat atgttgggaa gcgacgcatt ggatgacttt 2640
   gatctggaca tgctcggctc cgatgctctg gacgatttcg atctcgatat gttaattaac 2700
   tacccgtacg acgttccgga ctacgcttct tgagaattc 2739
<210> 40
   <211> 910
   <212> PRT
   <213> unknown
<220>
   <223> synthesized; polypeptide E2C-ER-L-ER-VP64
<400> 40
   Gly Ser Ala Thr Met Ala Gln Ala Ala Leu Glu Pro Gly Glu Lys Pro
   1 5 10 15
   Tyr Ala Cys Pro Glu Cys Gly Lys Ser Phe Ser Arg Lys Asp Ser Leu
   20 25 30
   Val Arg His Gln Arg Thr His Thr Gly Glu Lys Pro Tyr Lys Cys Pro
   35 40 45
   Glu Cys Gly Lys Ser Phe Ser Gln Ser Gly Asp Leu Arg Arg His Gln
   50 55 60
   Arg Thr His Thr Gly Glu Lys Pro Tyr Lys Cys Pro Glu Cys Gly Lys
   65 70 75 80
   Ser Phe Ser Asp Cys Arg Asp Leu Ala Arg His Gln Arg Thr His Thr
   85 90 95
   Gly Glu Lys Pro Tyr Ala Cys Pro Glu Cys Gly Lys Ser Phe Ser Gln
   100 105 110
   Ser Ser His Leu Val Arg His Gln Arg Thr His Thr Gly Glu Lys Pro
   115 120 125
   Tyr Lys Cys Pro Glu Cys Gly Lys Ser Phe Ser Asp Cys Arg Asp Leu
   130 135 140
   Ala Arg His Gln Arg Thr His Thr Gly Glu Lys Pro Tyr Lys Cys Pro
   145 150 155 160
   Glu Cys Gly Lys Ser Phe Ser Arg Ser Asp Lys Leu Val Arg His Gln
   165 170 175
   Arg Thr His Thr Gly Lys Lys Thr Ser Gly Gln Ala Gly Arg Arg Asn
   180 185 190
   Glu Met Gly Ala Ser Gly Asp Met Arg Ala Ala Asn Leu Trp Pro Ser
   195 200 205
   Pro Leu Val Ile Lys His Thr Lys Lys Asn Ser Pro Ala Leu Ser Leu
   210 215 220
   Thr Ala Asp Gln Met Val Ser Ala Leu Leu Asp Ala Glu Pro Pro Met
   225 230 235 240
   Ile Tyr Ser Glu Tyr Asp Pro Ser Arg Pro Phe Ser Glu Ala Ser Met
   245 250 255
   Met Gly Leu Leu Thr Asn Leu Ala Asp Arg Glu Leu Val His Met Ile
   260 265 270
   Asn Trp Ala Lys Arg Val Pro Gly Phe Gly Asp Leu Asn Leu His Asp
   275 280 285
   Gln Val His Leu Leu Glu Cys Ala Trp Leu Glu Ile Leu Met Ile Gly
   290 295 300
   Leu Val Trp Arg Ser Met Glu His Pro Gly Lys Leu Leu Phe Ala Pro
   305 310 315 320
   Asn Leu Leu Leu Asp Arg Asn Gln Gly Lys Cys Val Glu Gly Met Val
   325 330 335
   Glu Ile Phe Asp Met Leu Leu Ala Thr Ser Ser Arg Phe Arg Met Met
   340 345 350
   Asn Leu Gln Gly Glu Glu Phe Val Cys Leu Lys Ser Ile Ile Leu Leu
   355 360 365
   Asn Ser Gly Val Tyr Thr Phe Leu Ser Ser Thr Leu Lys Ser Leu Glu
   370 375 380
   Glu Lys Asp His Ile His Arg Val Leu Asp Lys Ile Thr Asp Thr Leu
   385 390 395 400
   Ile His Leu Met Ala Lys Ala Gly Leu Thr Leu Gln Gln Gln His Arg
   405 410 415
   Arg Leu Ala Gln Leu Leu Leu Ile Leu Ser His Ile Arg His Met Ser
   420 425 430
   Asn Lys Gly Met Glu His Leu Tyr Asn Met Lys Cys Lys Asn Val Val
   435 440 445
   Pro Leu Tyr Asp Leu Leu Leu Glu Met Leu Asp Ala His Arg Leu His
   450 455 460
   Ala Pro Ala Ser Arg Met Gly Val Pro Pro Glu Glu Pro Ser Gln Thr
   465 470 475 480
   Gln Leu Ala Thr Thr Ser Ser Thr Ser Ala His Ser Leu Gln Thr Tyr
   485 490 495
   Tyr Ile Pro Pro Glu Ala Glu Gly Phe Pro Asn Thr Ile Ser Ser Gly
   500 505 510
   Gly Gly Gly Ser Gly Gly Gly Gly Gly Gly Ser Thr Ser Ser Ser Asn
   515 520 525
   Glu Met Gly Ala Ser Gly Asp Met Arg Ala Ala Asn Leu Trp Pro Ser
   530 535 540
   Pro Leu Val Ile Lys His Thr Lys Lys Asn Ser Pro Ala Leu Ser Leu
   545 550 555 560
   Thr Ala Asp Gln Met Val Ser Ala Leu Leu Asp Ala Glu Pro Pro Met
   565 570 575
   Ile Tyr Ser Glu Tyr Asp Pro Ser Arg Pro Phe Ser Glu Ala Ser Met
   580 585 590
   Met Gly Leu Leu Thr Asn Leu Ala Asp Arg Glu Leu Val His Met Ile
   595 600 605
   Asn Trp Ala Lys Arg Val Pro Gly Phe Gly Asp Leu Asn Leu His Asp
   610 615 620
   Gln Val His Leu Leu Glu Cys Ala Trp Leu Glu Ile Leu Met Ile Gly
   625 630 635 640
   Leu Val Trp Arg Ser Met Glu His Pro Gly Lys Leu Leu Phe Ala Pro
   645 650 655
   Asn Leu Leu Leu Asp Arg Asn Gln Gly Lys Cys Val Glu Gly Met Val
   660 665 670
   Glu Ile Phe Asp Met Leu Leu Ala Thr Ser Ser Arg Phe Arg Met Met
   675 680 685
   Asn Leu Gln Gly Glu Glu Phe Val Cys Leu Lys Ser Ile Ile Leu Leu
   690 695 700
   Asn Ser Gly Val Tyr Thr Phe Leu Ser Ser Thr Leu Lys Ser Leu Glu
   705 710 715 720
   Glu Lys Asp His Ile His Arg Val Leu Asp Lys Ile Thr Asp Thr Leu
   725 730 735
   Ile His Leu Met Ala Lys Ala Gly Leu Thr Leu Gln Gln Gln His Arg
   740 745 750
   Arg Leu Ala Gln Leu Leu Leu Ile Leu Ser His Ile Arg His Met Ser
   755 760 765
   Asn Lys Gly Met Glu His Leu Tyr Asn Met Lys Cys Lys Asn Val Val
                   770                 775                 780
   Pro Leu Tyr Asp Leu Leu Leu Glu Met Leu Asp Ala His Arg Leu His
   785                 790                 795                 800
   Ala Pro Ala Ser Arg Met Gly Val Pro Pro Glu Glu Pro Ser Gln Thr
                   805                 810                 815
   Gln Leu Ala Thr Thr Ser Ser Thr Ser Ala His Ser Leu Gln Thr Tyr
               820                 825                 830
   Tyr Ile Pro Pro Glu Ala Glu Gly Phe Pro Asn Thr Ile Gly Arg Ala
           835                 840                 845
   Asp Ala Leu Asp Asp Phe Asp Leu Asp Met Leu Gly Ser Asp Ala Leu
       850                 855                 860
   Asp Asp Phe Asp Leu Asp Met Leu Gly Ser Asp Ala Leu Asp Asp Phe
   865                 870                 875                 880
   Asp Leu Asp Met Leu Gly Ser Asp Ala Leu Asp Asp Phe Asp Leu Asp
                   885                 890                 895
   Met Leu Ile Asn Tyr Pro Tyr Asp Val Pro Asp Tyr Ala Ser
               900                 905                 910
<210> 41
   <211> 2775
   <212> DNA
   <213> Unknown
<220>
   <223> synthesized; polypeptide E2C-ER-LL-ER-VP64
<400> 41
   ggatccgcca ccatggccca ggcggccctc gagcccgggg agaagcccta tgcttgtccg 60
   gaatgtggta agtccttcag taggaaggat tcgcttgtga ggcaccagcg tacccacacg 120
   ggtgaaaaac cgtataaatg cccagagtgc ggcaaatctt ttagtcagtc gggggatctt 180
   aggcgtcatc aacgcactca tactggcgag aagccataca aatgtccaga atgtggcaag 240
   tctttcagtg attgtcgtga tcttgcgagg caccaacgta ctcacaccgg ggagaagccc 300
   tatgcttgtc cggaatgtgg taagtccttc tctcagagct ctcacctggt gcgccaccag 360
   cgtacccaca cgggtgaaaa accgtataaa tgcccagagt gcggcaaatc ttttagtgac 420
   tgccgcgacc ttgctcgcca tcaacgcact catactggcg agaagccata caaatgtcca 480
   gaatgtggca agtctttcag ccgctctgac aagctggtgc gtcaccaacg tactcacacc 540
   ggtaaaaaaa ctagtggcca ggccggccgc cgaaatgaaa tgggtgcttc aggagacatg 600
   agggctgcca acctttggcc aagccctctt gtgattaagc acactaagaa gaatagccct 660
   gccttgtcct tgacagctga ccagatggtc agtgccttgt tggatgctga accgcccatg 720
   atctattctg aatatgatcc ttctagaccc ttcagtgaag cctcaatgat gggcttattg 780
   accaacctag cagataggga gctggttcat atgatcaact gggcaaagag agtgccaggc 840
   tttggggact tgaatctcca tgatcaggtc caccttctcg agtgtgcctg gctggagatt 900
   ctgatgattg gtctcgtctg gcgctccatg gaacacccgg ggaagctcct gtttgctcct 960
   aacttgctcc tggacaggaa tcaaggtaaa tgtgtggaag gcatggtgga gatctttgac 1020
   atgttgcttg ctacgtcaag tcggttccgc atgatgaacc tgcagggtga agagtttgtg 1080
   tgcctcaaat ccatcatttt gcttaattcc ggagtgtaca cgtttctgtc cagcaccttg 1140
   aagtctctgg aagagaagga ccacatccac cgtgtcctgg acaagatcac agacactttg 1200
   atccacctga tggccaaagc tggcctgact ctgcagcagc agcatcgccg cctagctcag 1260
   ctccttctca ttctttccca tatccggcac atgagtaaca aaggcatgga gcatctctac 1320
   aacatgaaat gcaagaacgt tgtgcccctc tatgacctgc tcctggagat gttggatgcc 1380
   caccgccttc atgccccagc cagtcgcatg ggagtgcccc cagaggagcc cagccagacc 1440
   cagctggcca ccaccagctc cacttcagca cattccttac aaacctacta catacccccg 1500
   gaagcagagg gcttccccaa cacgatctcc tctggtggcg gtggctcggg cggtggtggg 1560
   ggtggttcca ctagcggcgg tggcggtggc tcctctggtg gcggtggcgg ttcttccaat 1620
   gaaatgggtg cttcaggaga catgagggct gccaaccttt ggccaagccc tcttgtgatt 1680
   aagcacacta agaagaatag ccctgccttg tccttgacag ctgaccagat ggtcagtgcc 1740
   ttgttggatg ctgaaccgcc catgatctat tctgaatatg atccttctag acccttcagt 1800
   gaagcctcaa tgatgggctt attgaccaac ctagcagata gggagctggt tcatatgatc 1860
   aactgggcaa agagagtgcc aggctttggg gacttgaatc tccatgatca ggtccacctt 1920
   ctcgagtgtg cctggctgga gattctgatg attggtctcg tctggcgctc catggaacac 1980
   ccggggaagc tcctgtttgc tcctaacttg ctcctggaca ggaatcaagg taaatgtgtg 2040
   gaaggcatgg tggagatctt tgacatgttg cttgctacgt caagtcggtt ccgcatgatg 2100
   aacctgcagg gtgaagagtt tgtgtgcctc aaatccatca ttttgcttaa ttccggagtg 2160
   tacacgtttc tgtccagcac cttgaagtct ctggaagaga aggaccacat ccaccgtgtc 2220
   ctggacaaga tcacagacac tttgatccac ctgatggcca aagctggcct gactctgcag 2280
   cagcagcatc gccgcctagc tcagctcctt ctcattcttt cccatatccg gcacatgagt 2340
   aacaaaggca tggagcatct ctacaacatg aaatgcaaga acgttgtgcc cctctatgac 2400
   ctgctcctgg agatgttgga tgcccaccgc cttcatgccc cagccagtcg catgggagtg 2460
   cccccagagg agcccagcca gacccagctg gccaccacca gctccacttc agcacattcc 2520
   ttacaaacct actacatacc cccggaagca gagggcttcc ccaacacgat cgggcgcgcc 2580
   gacgcgctgg acgatttcga tctcgacatg ctgggttctg atgccctcga tgactttgac 2640
   ctggatatgt tgggaagcga cgcattggat gactttgatc tggacatgct cggctccgat 2700
   gctctggacg atttcgatct cgatatgtta attaactacc cgtacgacgt tccggactac 2760
   gcttcttgag aattc                                                  2775
<210> 42
   <211> 922
   <212> PRT
   <213> unknown
<220>
   <223> synthesized; polypeptide E2C-ER-LL-ER-VP64
<400> 42
   Gly Ser Ala Thr Met Ala Gln Ala Ala Leu Glu Pro Gly Glu Lys Pro
   1               5                   10                  15
   Tyr Ala Cys Pro Glu Cys Gly Lys Ser Phe Ser Arg Lys Asp Ser Leu
               20                  25                  30
   Val Arg His Gln Arg Thr His Thr Gly Glu Lys Pro Tyr Lys Cys Pro
           35                  40                  45
   Glu Cys Gly Lys Ser Phe Ser Gln Ser Gly Asp Leu Arg Arg His Gln
       50                  55                  60
   Arg Thr His Thr Gly Glu Lys Pro Tyr Lys Cys Pro Glu Cys Gly Lys
   65                  70                  75                  80
   Ser Phe Ser Asp Cys Arg Asp Leu Ala Arg His Gln Arg Thr His Thr
                   85                  90                  95
   Gly Glu Lys Pro Tyr Ala Cys Pro Glu Cys Gly Lys Ser Phe Ser Gln
               100                 105                 110
   Ser Ser His Leu Val Arg His Gln Arg Thr His Thr Gly Glu Lys Pro
           115                 120                 125
   Tyr Lys Cys Pro Glu Cys Gly Lys Ser Phe Ser Asp Cys Arg Asp Leu
       130                 135                 140
   Ala Arg His Gln Arg Thr His Thr Gly Glu Lys Pro Tyr Lys Cys Pro
   145                 150                 155                 160
   Glu Cys Gly Lys Ser Phe Ser Arg Ser Asp Lys Leu Val Arg His Gln
                   165                 170                 175
   Arg Thr His Thr Gly Lys Lys Thr Ser Gly Gln Ala Gly Arg Arg Asn
               180                 185                 190
   Glu Met Gly Ala Ser Gly Asp Met Arg Ala Ala Asn Leu Trp Pro Ser
           195                 200                 205
   Pro Leu Val Ile Lys His Thr Lys Lys Asn Ser Pro Ala Leu Ser Leu
       210                 215                 220
   Thr Ala Asp Gln Met Val Ser Ala Leu Leu Asp Ala Glu Pro Pro Met
   225                 230                 235                 240
   Ile Tyr Ser Glu Tyr Asp Pro Ser Arg Pro Phe Ser Glu Ala Ser Met
                   245                 250                 255
   Met Gly Leu Leu Thr Asn Leu Ala Asp Arg Glu Leu Val His Met Ile
               260                 265                 270
   Asn Trp Ala Lys Arg Val Pro Gly Phe Gly Asp Leu Asn Leu His Asp
           275                 280                 285
   Gln Val His Leu Leu Glu Cys Ala Trp Leu Glu Ile Leu Met Ile Gly
       290                 295                 300
   Leu Val Trp Arg Ser Met Glu His Pro Gly Lys Leu Leu Phe Ala Pro
   305                 310                 315                 320
   Asn Leu Leu Leu Asp Arg Asn Gln Gly Lys Cys Val Glu Gly Met Val
   325 330 335
   Glu Ile Phe Asp Met Leu Leu Ala Thr Ser Ser Arg Phe Arg Met Met
   340 345 350
   Asn Leu Gln Gly Glu Glu Phe Val Cys Leu Lys Ser Ile Ile Leu Leu
   355 360 365
   Asn Ser Gly Val Tyr Thr Phe Leu Ser Ser Thr Leu Lys Ser Leu Glu
   370 375 380
   Glu Lys Asp His Ile His Arg Val Leu Asp Lys Ile Thr Asp Thr Leu
   385 390 395 400
   Ile His Leu Met Ala Lys Ala Gly Leu Thr Leu Gln Gln Gln His Arg
   405 410 415
   Arg Leu Ala Gln Leu Leu Leu Ile Leu Ser His Ile Arg His Met Ser
   420 425 430
   Asn Lys Gly Met Glu His Leu Tyr Asn Met Lys Cys Lys Asn Val Val
   435 440 445
   Pro Leu Tyr Asp Leu Leu Leu Glu Met Leu Asp Ala His Arg Leu His
   450 455 460
   Ala Pro Ala Ser Arg Met Gly Val Pro Pro Glu Glu Pro Ser Gln Thr
   465 470 475 480
   Gln Leu Ala Thr Thr Ser Ser Thr Ser Ala His Ser Leu Gln Thr Tyr
   485 490 495
   Tyr Ile Pro Pro Glu Ala Glu Gly Phe Pro Asn Thr Ile Ser Ser Gly
   500 505 510
   Gly Gly Gly Ser Gly Gly Gly Gly Gly Gly Ser Thr Ser Gly Gly Gly
   515 520 525
   Gly Gly Ser Ser Gly Gly Gly Gly Gly Ser Ser Asn Glu Met Gly Ala
   530 535 540
   Ser Gly Asp Met Arg Ala Ala Asn Leu Trp Pro Ser Pro Leu Val Ile
   545 550 555 560
   Lys His Thr Lys Lys Asn Ser Pro Ala Leu Ser Leu Thr Ala Asp Gln
   565 570 575
   Met Val Ser Ala Leu Leu Asp Ala Glu Pro Pro Met Ile Tyr Ser Glu
   580 585 590
   Tyr Asp Pro Ser Arg Pro Phe Ser Glu Ala Ser Met Met Gly Leu Leu
   595 600 605
   Thr Asn Leu Ala Asp Arg Glu Leu Val His Met Ile Asn Trp Ala Lys
   610 615 620
   Arg Val Pro Gly Phe Gly Asp Leu Asn Leu His Asp Gln Val His Leu
   625 630 635 640
   Leu Glu Cys Ala Trp Leu Glu Ile Leu Met Ile Gly Leu Val Trp Arg
   645 650 655
   Ser Met Glu His Pro Gly Lys Leu Leu Phe Ala Pro Asn Leu Leu Leu
   660 665 670
   Asp Arg Asn Gln Gly Lys Cys Val Glu Gly Met Val Glu Ile Phe Asp
   675 680 685
   Met Leu Leu Ala Thr Ser Ser Arg Phe Arg Met Met Asn Leu Gln Gly
   690 695 700
   Glu Glu Phe Val Cys Leu Lys Ser Ile Ile Leu Leu Asn Ser Gly Val
   705 710 715 720
   Tyr Thr Phe Leu Ser Ser Thr Leu Lys Ser Leu Glu Glu Lys Asp His
   725 730 735
   Ile His Arg Val Leu Asp Lys Ile Thr Asp Thr Leu Ile His Leu Met
   740 745 750
   Ala Lys Ala Gly Leu Thr Leu Gln Gln Gln His Arg Arg Leu Ala Gln
   755 760 765
   Leu Leu Leu Ile Leu Ser His Ile Arg His Met Ser Asn Lys Gly Met
   770 775 780
   Glu His Leu Tyr Asn Met Lys Cys Lys Asn Val Val Pro Leu Tyr Asp
   785 790 795 800
   Leu Leu Leu Glu Met Leu Asp Ala His Arg Leu His Ala Pro Ala Ser
   805 810 815
   Arg Met Gly Val Pro Pro Glu Glu Pro Ser Gln Thr Gln Leu Ala Thr
   820 825 830
   Thr Ser Ser Thr Ser Ala His Ser Leu Gln Thr Tyr Tyr Ile Pro Pro
           835                 840                 845
   Glu Ala Glu Gly Phe Pro Asn Thr Ile Gly Arg Ala Asp Ala Leu Asp
       850                 855                 860
   Asp Phe Asp Leu Asp Met Leu Gly Ser Asp Ala Leu Asp Asp Phe Asp
   865                 870                 875                 880
   Leu Asp Met Leu Gly Ser Asp Ala Leu Asp Asp Phe Asp Leu Asp Met
                   885                 890                 895
   Leu Gly Ser Asp Ala Leu Asp Asp Phe Asp Leu Asp Met Leu Ile Asn
               900                 905                 910
   Tyr Pro Tyr Asp Val Pro Asp Tyr Ala Ser
           915                 920
<210> 43
   <211> 2775
   <212> DNA
   <213> Unknown
<220>
   <223> Synthesized; complementary sequence to SEQ ID 41
<400> 43
   gaattctcaa gaagcgtagt ccggaacgtc gtacgggtag ttaattaaca tatcgagatc 60
   gaaatcgtcc agagcatcgg agccgagcat gtccagatca aagtcatcca atgcgtcgct 120
   tcccaacata tccaggtcaa agtcatcgag ggcatcagaa cccagcatgt cgagatcgaa 180
   atcgtccagc gcgtcggcgc gcccgatcgt gttggggaag ccctctgctt ccgggggtat 240
   gtagtaggtt tgtaaggaat gtgctgaagt ggagctggtg gtggccagct gggtctggct 300
   gggctcctct gggggcactc ccatgcgact ggctggggca tgaaggcggt gggcatccaa 360
   catctccagg agcaggtcat agaggggcac aacgttcttg catttcatgt tgtagagatg 420
   ctccatgcct ttgttactca tgtgccggat atgggaaaga atgagaagga gctgagctag 480
   gcggcgatgc tgctgctgca gagtcaggcc agctttggcc atcaggtgga tcaaagtgtc 540
   tgtgatcttg tccaggacac ggtggatgtg gtccttctct tccagagact tcaaggtgct 600
   ggacagaaac gtgtacactc cggaattaag caaaatgatg gatttgaggc acacaaactc 660
   ttcaccctgc aggttcatca tgcggaaccg acttgacgta gcaagcaaca tgtcaaagat 720
   ctccaccatg ccttccacac atttaccttg attcctgtcc aggagcaagt taggagcaaa 780
   caggagcttc cccgggtgtt ccatggagcg ccagacgaga ccaatcatca gaatctccag 840
   ccaggcacac tcgagaaggt ggacctgatc atggagattc aagtccccaa agcctggcac 900
   tctctttgcc cagttgatca tatgaaccag ctccctatct gctaggttgg tcaataagcc 960
   catcattgag gcttcactga agggtctaga aggatcatat tcagaataga tcatgggcgg 1020
   ttcagcatcc aacaaggcac tgaccatctg gtcagctgtc aaggacaagg cagggctatt 1080
   cttcttagtg tgcttaatca caagagggct tggccaaagg ttggcagccc tcatgtctcc 1140
   tgaagcaccc atttcattgg aagaaccgcc accgccacca gaggagccac cgccaccgcc 1200
   gctagtggaa ccacccccac caccgcccga gccaccgcca ccagaggaga tcgtgttggg 1260
   gaagccctct gcttccgggg gtatgtagta ggtttgtaag gaatgtgctg aagtggagct 1320
   ggtggtggcc agctgggtct ggctgggctc ctctgggggc actcccatgc gactggctgg 1380
   ggcatgaagg cggtgggcat ccaacatctc caggagcagg tcatagaggg gcacaacgtt 1440
   cttgcatttc atgttgtaga gatgctccat gcctttgtta ctcatgtgcc ggatatggga 1500
   aagaatgaga aggagctgag ctaggcggcg atgctgctgc tgcagagtca ggccagcttt 1560
   ggccatcagg tggatcaaag tgtctgtgat cttgtccagg acacggtgga tgtggtcctt 1620
   ctcttccaga gacttcaagg tgctggacag aaacgtgtac actccggaat taagcaaaat 1680
   gatggatttg aggcacacaa actcttcacc ctgcaggttc atcatgcgga accgacttga 1740
   cgtagcaagc aacatgtcaa agatctccac catgccttcc acacatttac cttgattcct 1800
   gtccaggagc aagttaggag caaacaggag cttccccggg tgttccatgg agcgccagac 1860
   gagaccaatc atcagaatct ccagccaggc acactcgaga aggtggacct gatcatggag 1920
   attcaagtcc ccaaagcctg gcactctctt tgcccagttg atcatatgaa ccagctccct 1980
   atctgctagg ttggtcaata agcccatcat tgaggcttca ctgaagggtc tagaaggatc 2040
   atattcagaa tagatcatgg gcggttcagc atccaacaag gcactgacca tctggtcagc 2100
   tgtcaaggac aaggcagggc tattcttctt agtgtgctta atcacaagag ggcttggcca 2160
   aaggttggca gccctcatgt ctcctgaagc acccatttca tttcggcggc cggcctggcc 2220
   actagttttt ttaccggtgt gagtacgttg gtgacgcacc agcttgtcag agcggctgaa 2280
   agacttgcca cattctggac atttgtatgg cttctcgcca gtatgagtgc gttgatggcg 2340
   agcaaggtcg cggcagtcac taaaagattt gccgcactct gggcatttat acggtttttc 2400
   acccgtgtgg gtacgctggt ggcgcaccag gtgagagctc tgagagaagg acttaccaca 2460
   ttccggacaa gcatagggct tctccccggt gtgagtacgt tggtgcctcg caagatcacg 2520
   acaatcactg aaagacttgc cacattctgg acatttgtat ggcttctcgc cagtatgagt 2580
   gcgttgatga cgcctaagat cccccgactg actaaaagat ttgccgcact ctgggcattt 2640
   atacggtttt tcacccgtgt gggtacgctg gtgcctcaca agcgaatcct tcctactgaa 2700
   ggacttacca cattccggac aagcataggg cttctccccg ggctcgaggg ccgcctgggc 2760
   catggtggcg gatcc                                                  2775
<210> 44
   <211> 2739
   <212> DNA
   <213> Unknown
<220>
   <223> Synthesized; complementary sequence to SEQ ID 39
<400> 44
   gaattctcaa gaagcgtagt ccggaacgtc gtacgggtag ttaattaaca tatcgagatc 60
   gaaatcgtcc agagcatcgg agccgagcat gtccagatca aagtcatcca atgcgtcgct 120
   tcccaacata tccaggtcaa agtcatcgag ggcatcagaa cccagcatgt cgagatcgaa 180
   atcgtccagc gcgtcggcgc gcccgatcgt gttggggaag ccctctgctt ccgggggtat 240
   gtagtaggtt tgtaaggaat gtgctgaagt ggagctggtg gtggccagct gggtctggct 300
   gggctcctct gggggcactc ccatgcgact ggctggggca tgaaggcggt gggcatccaa 360
   catctccagg agcaggtcat agaggggcac aacgttcttg catttcatgt tgtagagatg 420
   ctccatgcct ttgttactca tgtgccggat atgggaaaga atgagaagga gctgagctag 480
   gcggcgatgc tgctgctgca gagtcaggcc agctttggcc atcaggtgga tcaaagtgtc 540
   tgtgatcttg tccaggacac ggtggatgtg gtccttctct tccagagact tcaaggtgct 600
   ggacagaaac gtgtacactc cggaattaag caaaatgatg gatttgaggc acacaaactc 660
   ttcaccctgc aggttcatca tgcggaaccg acttgacgta gcaagcaaca tgtcaaagat 720
   ctccaccatg ccttccacac atttaccttg attcctgtcc aggagcaagt taggagcaaa 780
   caggagcttc cccgggtgtt ccatggagcg ccagacgaga ccaatcatca gaatctccag 840
   ccaggcacac tcgagaaggt ggacctgatc atggagattc aagtccccaa agcctggcac 900
   tctctttgcc cagttgatca tatgaaccag ctccctatct gctaggttgg tcaataagcc 960
   catcattgag gcttcactga agggtctaga aggatcatat tcagaataga tcatgggcgg 1020
   ttcagcatcc aacaaggcac tgaccatctg gtcagctgtc aaggacaagg cagggctatt 1080
   cttcttagtg tgcttaatca caagagggct tggccaaagg ttggcagccc tcatgtctcc 1140
   tgaagcaccc atttcattgg aagagctagt ggaaccaccc ccaccaccgc ccgagccacc 1200
   gccaccagag gagatcgtgt tggggaagcc ctctgcttcc gggggtatgt agtaggtttg 1260
   taaggaatgt gctgaagtgg agctggtggt ggccagctgg gtctggctgg gctcctctgg 1320
   gggcactccc atgcgactgg ctggggcatg aaggcggtgg gcatccaaca tctccaggag 1380
   caggtcatag aggggcacaa cgttcttgca tttcatgttg tagagatgct ccatgccttt 1440
   gttactcatg tgccggatat gggaaagaat gagaaggagc tgagctaggc ggcgatgctg 1500
   ctgctgcaga gtcaggccag ctttggccat caggtggatc aaagtgtctg tgatcttgtc 1560
   caggacacgg tggatgtggt ccttctcttc cagagacttc aaggtgctgg acagaaacgt 1620
   gtacactccg gaattaagca aaatgatgga tttgaggcac acaaactctt caccctgcag 1680
   gttcatcatg cggaaccgac ttgacgtagc aagcaacatg tcaaagatct ccaccatgcc 1740
   ttccacacat ttaccttgat tcctgtccag gagcaagtta ggagcaaaca ggagcttccc 1800
   cgggtgttcc atggagcgcc agacgagacc aatcatcaga atctccagcc aggcacactc 1860
   gagaaggtgg acctgatcat ggagattcaa gtccccaaag cctggcactc tctttgccca 1920
   gttgatcata tgaaccagct ccctatctgc taggttggtc aataagccca tcattgaggc 1980
   ttcactgaag ggtctagaag gatcatattc agaatagatc atgggcggtt cagcatccaa 2040
   caaggcactg accatctggt cagctgtcaa ggacaaggca gggctattct tcttagtgtg 2100
   cttaatcaca agagggcttg gccaaaggtt ggcagccctc atgtctcctg aagcacccat 2160
   ttcatttcgg cggccggcct ggccactagt ttttttaccg gtgtgagtac gttggtgacg 2220
   caccagcttg tcagagcggc tgaaagactt gccacattct ggacatttgt atggcttctc 2280
   gccagtatga gtgcgttgat ggcgagcaag gtcgcggcag tcactaaaag atttgccgca 2340
   ctctgggcat ttatacggtt tttcacccgt gtgggtacgc tggtggcgca ccaggtgaga 2400
   gctctgagag aaggacttac cacattccgg acaagcatag ggcttctccc cggtgtgagt 2460
   acgttggtgc ctcgcaagat cacgacaatc actgaaagac ttgccacatt ctggacattt 2520
   gtatggcttc tcgccagtat gagtgcgttg atgacgccta agatcccccg actgactaaa 2580
   agatttgccg cactctgggc atttatacgg tttttcaccc gtgtgggtac gctggtgcct 2640
   cacaagcgaa tccttcctac tgaaggactt accacattcc ggacaagcat agggcttctc 2700
   cccgggctcg agggccgcct gggccatggt ggcggatcc                        2739
<210> 45
   <211> 558
   <212> DNA
   <213> Unknown
<220>
   <223> Synthesized; complementary sequence to SEQ ID 35
<221> misc_feature
   <222> 1, 2, 557, 558
   <223> n = A,T,C or G
<400> 45
   nnggccggcc tggccactag tttttttacc ggtgtgagta cgttggtggc gaaccaggtg 60
   gctagactgg gagaaagact tgccacattc tggacatttg tatggcttct cgccagtatg 120
   agtgcgttga tggcgcacca gtttatcgga gcgactaaaa gatttgccgc actctgggca 180
   tttatacggt ttttcacccg tgtgggtacg ctggtggcga accaggttgc caggatcact 240
   gaaggactta ccacattccg gacaagcata gggcttctcc ccggtgtgag tacgttggtg 300
   acggaccaag tcgtctgaac gtgagaaaga cttgccacat tctggacatt tgtatggctt 360
   ctcgccagta tgagtgcgtt gatggcgcac cagcacatcg ctgcgactaa aagatttgcc 420
   gcactctggg catttatacg gtttttcacc cgtgtgggta cgctggtggc gcaccaggtg 480
   gccactggtg ctgaaggact taccacattc cggacaagca tagggcttct ccccgggctc 540
   gagggccgcc tgggccnn 558
<210> 46
   <211> 567
   <212> DNA
   <213> unknown
<220>
   <223> Synthesized; complementary sequence to SEQ ID 33
<221> misc_feature
   <222> 1, 2, 566, 567
   <223> n = A,T,C or G
<400> 46
   nnggccggcc tggccactag ttctagagtc cttctgtctt aaatggattt tggtatgcct 60
   cttgcgttca tcactcctgg caaacttcct cccacaaatg tcacaggcaa aaggcttctc 120
   gcctgtgtgg gtgcggatgt gggtggtaag gtggtcacta cgactgaagt tacgcatgca 180
   tattcgacac tggaagggct tctggcctgt gtggatgcgg atatggcgct tcagatcagc 240
   cgacttagaa aagcggcgat cgcaggactc gacagggcaa gcatagggct tctcaccggt 300
   atggattttg gtatgcctct tgcgttcatc actcctggca aacttcctcc cacaaatgtc 360
   acaggcaaaa ggcttctcgc ctgtgtgggt gcggatgtgg gtggtaaggt ggtcactacg 420
   actgaagtta cgcatgcata ttcgacactg gaagggcttc tggcctgtgt ggatgcggat 480
   atggcgcttc agatcagccg acttagaaaa gcggcgatcg caggactcga cagggcaagc 540
   atagggctcg agggccgcct gggccnn 567
<210> 47
   <211> 558
   <212> DNA
   <213> Unknown
<220>
   <223> Synthesized; complementary sequence to SEQ ID 31
   <221> misc_feature
   <222> 1, 2, 557, 558
   <223> n = A,T,C or G
<400> 47
   nnggccggcc tggccactag tttttttacc ggtgtgagta cgttggtgcc tcgcaagatc 60
   acgacaatca ctgaaagact tgccacattc tggacatttg tatggcttct cgccagtatg 120
   agtgcgttga tggctggcca ggtggccggc ctggctaaaa gatttgccgc actctgggca 180
   tttatacggt ttttcacccg tgtgggtacg ctggtggcgc accaggttat cgctgcggct 240
   gaaggactta ccacattccg gacaagcata gggcttctcc ccggtgtgag tacgttggtg 300
   gcgaaccagg tggctagact gggagaaaga cttgccacat tctggacatt tgtatggctt 360
   ctcgccagta tgagtgcgtt gatggcgaac cagatcatcg ctgcggctaa aagatttgcc 420
   gcactctggg catttatacg gtttttcacc cgtgtgggta cgctggtggc gcaccagcac 480
   atcgctgcga ctgaaggact taccacattc cggacaagca tagggcttct ccccgggctc 540
   gagggccgcc tgggccnn                                               558
<210> 48
   <211> 38
   <212> PRT
   <213> Unknown
<220>
   <223> synthesized; peptide B3/F1
<400> 48
   Ala Gln Ala Ala Leu Glu Pro Lys Glu Lys Pro Tyr Ala Cys Pro Glu
    1               5                  10                  15
   Cys Gly Lys Ser Phe Ser Asp Pro Gly Asn Leu Val Arg His Gln Arg
               20                  25                  30
   Thr His Thr Gly Glu Lys
           35
<210> 49
   <211> 28
   <212> PRT
   <213> Unknown
<220>
   <223> synthesized; peptide B3/F2
<400> 49
   Pro Tyr Lys Cys Pro Glu Cys Gly Lys Ser Phe Ser Arg Ser Asp Lys
    1               5                  10                  15
   Leu Val Arg His Gln Arg Thr His Thr Gly Glu Lys
               20                  25
<210> 50
   <211> 34
   <212> PRT
   <213> unknown
<220>
   <223> synthesized; peptide B3/F3
<400> 50
   Pro Tyr Lys Cys Pro Glu Cys Gly Lys Ser Phe Ser Gln Ser Ser His
   1               5                   10                  15
   Leu Val Arg His Gln Arg Thr His Thr Gly Lys Lys Thr Ser Gly Gln
               20                  25                  30
   Ala Gly
<210> 51
   <211> 38
   <212> PRT
   <213> unknown
<220>
   <223> synthesized; peptide N1/F1
<400> 51
   Ala Gln Ala Ala Leu Glu Pro Lys Glu Lys Pro Tyr Ala Cys Pro Glu
   1               5                   10                  15
   Cys Gly Lys Ser Phe Ser Gln Ser Ser Ser Leu Val Arg His Gln Arg
               20                  25                  30
   Thr His Thr Gly Glu Lys
           35
<210> 52
   <211> 28
   <212> PRT
   <213> Unknown
<220>
   <223> Synthesized; peptide N1/F2
<400> 52
   Pro Tyr Lys Cys Pro Glu Cys Gly Lys Ser Phe Ser Gln Ser Ser Asn
   1               5                   10                  15
   Leu Val Arg His Gln Arg Thr His Thr Gly Glu Lys
               20                  25
<210> 53
   <211> 34
   <212> PRT
   <213> Unknown
<220>
   <223> synthesized; peptide N1/F3
<400> 53
   Pro Tyr Lys Cys Pro Glu Cys Gly Lys Ser Phe Ser Arg Ser Asp Lys
   1               5                   10                  15
   Leu Val Arg His Gln Arg Thr His Thr Gly Lys Lys Thr Ser Gly Gln
               20                  25                  30
   Ala Gly